(19) Europäisches Patentamt
European Patent Office
Office européen des brevets

(11) EP 1 920 051 B1

(12) EUROPEAN PATENT SPECIFICATION

(45) Date of publication and mention of the grant of the patent:
**20.10.2010 Bulletin 2010/42**

(21) Application number: **06779418.0**

(22) Date of filing: **14.09.2006**

(51) Int Cl.:
*C12N 9/20* (2006.01)

(86) International application number:
**PCT/GB2006/003406**

(87) International publication number:
**WO 2008/032007 (20.03.2008 Gazette 2008/12)**

(54) **LIPASE**

LIPASE

LIPASE

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**14.05.2008 Bulletin 2008/20**

(73) Proprietor: **TMO Renewables Limited**
**Guildford, Surrey GU2 7YF (GB)**

(72) Inventors:
- **ATKINSON, Tony**
  **Salisbury**
  **Wiltshire SP4 6JL (GB)**
- **CRIPPS, Roger**
  **Malmesbury**
  **Wiltshire SN16 90T (GB)**
- **ELEY, Kirstin**
  **Guildford**
  **Surrey, GU1 4PF (GB)**
- **DANSON, Michael**
  **Bristol BS31 3AQ (GB)**
- **HOUGH, David W.**
  **Bath BA2 2AX (GB)**

(74) Representative: **Dean, John Paul et al**
**Withers & Rogers LLP**
**Goldings House**
**2 Hays Lane**
**London**
**SE1 2HW (GB)**

(56) References cited:
**EP-A- 0 384 717** **EP-A- 1 624 056**
**EP-A1- 0 872 548** **WO-A2-2004/067705**

- **CHO A-RA ET AL: "Cloning, sequencing and expression in Escherichia coli of a thermophilic lipase from Bacillus thermoleovorans ID-1" FEMS MICROBIOLOGY LETTERS, vol. 186, no. 2, 15 May 2000 (2000-05-15), pages 235-238, XP002417335 ISSN: 0378-1097 cited in the application**
- **LI H ET AL: "Characterization of thermostable lipase from thermophilic Geobacillus sp. TW1" PROTEIN EXPRESSION AND PURIFICATION, ACADEMIC PRESS, SAN DIEGO, CA, US, vol. 42, no. 1, July 2005 (2005-07), pages 153-159, XP004918459 ISSN: 1046-5928 cited in the application**
- **CHOR LEOW T ET AL: "High level expression of thermostable lipase from Geobacillus sp. strain T1" BIOSCIENCE BIOTECHNOLOGY BIOCHEMISTRY, JAPAN SOC. FOR BIOSCIENCE, BIOTECHNOLOGY AND AGROCHEM, TOKYO, JP, vol. 68, no. 1, January 2004 (2004-01), pages 96-103, XP002354934 ISSN: 0916-8451**
- **SUNNA ANWAR ET AL: "Biochemical characterization of a recombinant thermoalkalophilic lipase and assessment of its substrate enantioselectivity" ENZYME AND MICROBIAL TECHNOLOGY, vol. 31, no. 4, 2 September 2002 (2002-09-02), pages 472-476, XP002417336 ISSN: 0141-0229 cited in the application**
- **DATABASE UniProt [Online] Swissprot; 7 December 2004 (2004-12-07), RHAMAN, R.N.Z.R.A. ET AL.: "Thermostable organic solvent tolerant lipase" XP002417401 retrieved from EBI accession no. Q5U780 Database accession no. Q5U780_9BACI**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE UniProt [Online] Swissprot; 15 February 2005 (2005-02-15), RHAMAN, R.N.Z.A. ET AL.: "Thermostable lipase (EC 3.1.1.3)." XP002417402 retrieved from EBI accession no. Q5I4I3 Database accession no. Q5I4I3_9BACI
- DATABASE EMBL [Online] 25 November 2002 (2002-11-25), "Geobacillus thermoleovorans IHI-91 thermophilic lipase gene, complete cds." XP002417403 retrieved from EBI accession no. EM_PRO:AY149997 Database accession no. AY149997
- ISO MAMORU ET AL: "Production of biodiesel fuel from triglycerides and alcohol using immobilized lipase" JOURNAL OF MOLECULAR CATALYSIS B ENZYMATIC, vol. 16, no. 1, 20 November 2001 (2001-11-20), pages 53-58, XP002417337 ISSN: 1381-1177
- MODI MUKESH KUMAR ET AL: "Lipase-mediated transformation of vegetable oils into biodiesel using propan-2-ol as acyl acceptor" BIOTECHNOLOGY LETTERS, vol. 28, no. 9, May 2006 (2006-05), pages 637-640, XP002417338 ISSN: 0141-5492
- MARKOSSIAN SAMSON; BECKER PETER; MAERKL HERBERT; ANTRANIKIAN GARABED: "Isolation and characterization of lipid-degrading Bacillus thermoleovorans IHI-91 from an icelandic hot spring" EXTREMOPHILES, vol. 4, no. 6, December 2000 (2000-12), pages 365-371,

**SEQUENCE LISTING**

<110> TMO Renewables Ltd

<120> Lipase

<130> P105323PCT-1

<160> 15

<170> PatentIn version 3.3

<210> 1
<211> 34
<212> DNA
<213> primer sequence

<400> 1
aaaaaacggg atccagcggc agtttcacgc gcca 34

<210> 2
<211> 33
<212> DNA
<213> primer sequence

<400> 2
gataatccct aagcttaagg ccgcaaactc gcc 33

<210> 3
<211> 389
<212> PRT
<213> Bacillus OK1

<400> 3

```
Ala Ala Val Ser Arg Ala Asn Asp Ala Pro Ile Val Leu Leu His Gly
1               5                   10                  15

Phe Thr Gly Trp Gly Arg Glu Glu Met Phe Gly Phe Lys Tyr Trp Gly
            20                  25                  30

Gly Val Arg Gly Asp Ile Glu Gln Trp Leu Asn Asp Asn Gly Tyr Arg
        35                  40                  45

Thr Tyr Thr Leu Ala Val Gly Pro Leu Ser Ser Asn Trp Asp Arg Ala
    50                  55                  60
```

Cys Glu Ala Tyr Ala Gln Leu Val Gly Gly Thr Val Asp Tyr Gly Ala
65                  70                  75                  80

Ala His Ala Ala Lys His Gly His Ala Arg Phe Gly Arg Thr Tyr Pro
                85                  90                  95

Gly Leu Leu Pro Glu Leu Lys Arg Gly Gly Arg Ile His Ile Ile Ala
            100                 105                 110

His Ser Gln Gly Gly Gln Thr Ala Arg Met Leu Val Ser Leu Leu Glu
        115                 120                 125

Asn Gly Ser Gln Glu Glu Arg Glu Tyr Ala Lys Ala His Asn Val Ser
    130                 135                 140

Leu Ser Pro Leu Phe Glu Gly Gly His His Val Val Leu Ser Val Thr
145                 150                 155                 160

Thr Ile Ala Thr Pro His Asp Gly Thr Thr Leu Val Asn Met Val Asp
                165                 170                 175

Phe Thr Asp Arg Phe Phe Asp Leu Gln Lys Ala Val Leu Glu Ala Ala
            180                 185                 190

Ala Val Ala Ser Asn Val Pro Tyr Thr Ser Gln Val Tyr Asp Phe Lys
        195                 200                 205

Leu Asp Gln Trp Gly Leu Arg Arg Gln Pro Gly Glu Ser Phe Asp His
    210                 215                 220

Tyr Phe Glu Arg Leu Lys Arg Ser Pro Val Trp Thr Ser Thr Asp Thr
225                 230                 235                 240

Ala Arg Tyr Asp Leu Ser Val Ser Gly Ala Glu Lys Leu Asn Gln Trp
                245                 250                 255

Val Gln Ala Ser Pro Asn Thr Tyr Tyr Leu Ser Phe Ala Thr Glu Arg
            260                 265                 270

Thr Tyr Arg Gly Ala Leu Thr Gly Asn Tyr Tyr Pro Glu Leu Gly Met
        275                 280                 285

Asn Ala Phe Ser Ala Val Val Cys Ala Pro Phe Leu Gly Ser Tyr Arg
290 295 300

Asn Pro Thr Leu Gly Ile Asp Asp Arg Trp Leu Glu Asn Asp Gly Ile
305 310 315 320

Val Asn Thr Val Ser Met Asn Gly Pro Lys Arg Gly Ser Ser Asp Arg
325 330 335

Ile Val Pro Tyr Asp Gly Ala Leu Lys Lys Gly Val Trp Asn Asp Met
340 345 350

Gly Thr Tyr Asn Val Asp His Leu Glu Ile Ile Gly Val Asp Pro Asn
355 360 365

Pro Ser Phe Asp Ile Arg Ala Phe Tyr Leu Arg Leu Ala Glu Gln Leu
370 375 380

Ala Ser Leu Arg Pro
385

<210> 4
<211> 395
<212> PRT
<213> Bacillus F4b

<400> 4

Phe Lys Asn Gly Asp Pro Ala Ala Val Ser Arg Ala Asn Asp Ala Pro
1 5 10 15

Ile Val Leu Leu His Gly Phe Thr Gly Trp Gly Arg Glu Glu Met Phe
20 25 30

Gly Phe Lys Tyr Trp Gly Gly Val Arg Gly Asp Ile Glu Gln Trp Leu
35 40 45

Asn Asp Asn Gly Tyr Arg Thr Tyr Thr Leu Ala Val Gly Pro Leu Ser
50 55 60

Ser Asn Trp Asp Arg Ala Cys Glu Ala Tyr Ala Gln Leu Val Gly Gly
65 70 75 80

Thr Val Asp Tyr Gly Ala Ala His Ala Ala Lys His Gly His Ala Arg
85 90 95

Phe Gly Arg Thr Tyr Pro Gly Leu Leu Pro Glu Leu Lys Arg Gly Gly
100 105 110

Arg Val His Ile Ile Ala His Ser Gln Gly Gly Gln Thr Ala Arg Met
115 120 125

Leu Val Ser Leu Leu Glu Asn Gly Ser Gln Glu Glu Arg Glu Tyr Ala
130 135 140

Lys Glu His Asn Val Ser Leu Ser Pro Leu Phe Glu Gly Gly His Arg
145 150 155 160

Phe Val Leu Ser Val Thr Thr Ile Ala Thr Pro His Asp Gly Thr Thr
165 170 175

Leu Val Asn Met Val Asp Phe Thr Asp Arg Phe Phe Asp Leu Gln Lys
180 185 190

Ala Val Leu Glu Ala Ala Ala Val Ala Ser Asn Val Pro Tyr Thr Ser
195 200 205

Gln Val Tyr Asp Phe Lys Leu Asp Gln Trp Gly Leu Arg Arg Gln Pro
210 215 220

Gly Glu Ser Phe Asp His Tyr Phe Glu Arg Leu Lys Arg Ser Pro Val
225 230 235 240

Trp Thr Ser Thr Asp Thr Ala Arg Tyr Asp Leu Ser Val Ser Gly Ala
245 250 255

Glu Lys Leu Asn Gln Trp Val Gln Ala Ser Pro Asn Thr Tyr Tyr Leu
260 265 270

Ser Phe Ser Thr Glu Arg Thr Tyr Arg Gly Ala Leu Thr Gly Asn Tyr
275 280 285

Tyr Pro Glu Leu Gly Met Asn Ala Phe Ser Ala Val Val Cys Ala Pro
290 295 300

Phe Leu Gly Ser Tyr Arg Asn Pro Thr Leu Gly Ile Asp Asp Arg Trp
305 310 315 320

Leu Glu Asn Asp Gly Ile Val Asn Thr Val Ser Met Asn Gly Pro Lys
325 330 335

Arg Gly Ser Ser Asp Arg Ile Val Pro Tyr Asp Gly Ala Leu Lys Lys
340 345 350

Gly Val Trp Asn Asp Met Gly Thr Tyr Asn Val Asp His Leu Glu Ile
355 360 365

Ile Gly Val Asp Pro Asn Pro Ser Phe Asp Ile Arg Ala Phe Tyr Leu
370 375 380

Arg Leu Ala Glu Gln Leu Ala Ser Leu Arg Pro
385 390 395

<210> 5
<211> 389
<212> PRT
<213> Bacillus thermoleovorans

<400> 5

```
Ala Ala Val Ser Arg Ala Asn Asp Ala Pro Ile Val Leu Leu His Gly
1               5                   10                  15

Phe Thr Gly Trp Gly Arg Glu Glu Met Phe Gly Phe Lys Tyr Trp Gly
            20                  25                  30

Gly Val Arg Gly Asp Ile Glu Gln Trp Leu Asn Asp Asn Gly Tyr Arg
        35                  40                  45

Thr Tyr Thr Leu Ala Val Gly Pro Leu Ser Ser Asn Trp Asp Arg Ala
    50                  55                  60

Cys Glu Ala Tyr Ala Gln Leu Val Gly Gly Thr Val Asp Tyr Gly Ala
65                  70                  75                  80

Ala His Ala Ala Lys His Gly His Ala Arg Phe Gly Arg Thr Tyr Pro
                85                  90                  95
```

Gly Leu Leu Pro Glu Leu Lys Arg Gly Gly Arg Ile His Ile Ile Ala
100 105 110

His Ser Gln Gly Gly Gln Thr Ala Arg Met Leu Val Ser Leu Leu Glu
115 120 125

Asn Gly Ser Gln Glu Glu Arg Glu Tyr Ala Lys Ala His Asn Val Ser
130 135 140

Leu Ser Pro Leu Phe Glu Gly Gly His His Val Met Leu Ser Val Thr
145 150 155 160

Thr Ile Ala Thr Pro His Asp Gly Thr Thr Leu Val Asn Met Val Asp
165 170 175

Phe Thr Asp Arg Phe Phe Asp Leu Gln Lys Ala Val Leu Glu Ala Ala
180 185 190

Ala Val Ala Ser Asn Val Pro Tyr Thr Ser Gln Val Tyr Asp Phe Lys
195 200 205

Leu Asp Gln Trp Gly Leu Arg Arg Gln Pro Gly Glu Ser Phe Asp His
210 215 220

Tyr Phe Glu Arg Leu Lys Arg Ser Pro Val Trp Thr Ser Thr Asp Thr
225 230 235 240

Ala Arg Tyr Asp Leu Ser Val Ser Gly Ala Glu Lys Leu Asn Gln Trp
245 250 255

Val Gln Ala Ser Pro Asn Thr Tyr Tyr Leu Ser Phe Ala Thr Glu Arg
260 265 270

Thr Tyr Arg Gly Ala Leu Thr Gly Asn Tyr Tyr Pro Glu Leu Gly Met
275 280 285

Asn Ala Phe Ser Ala Val Val Cys Ala Pro Phe Leu Gly Ser Tyr Arg
290 295 300

Asn Pro Thr Leu Gly Ile Asp Asp Arg Trp Leu Glu Asn Asp Gly Ile
305 310 315 320

Val Asn Thr Val Ser Met Asn Gly Pro Lys Arg Gly Ser Ser Asp Arg
325 330 335

Ile Val Pro Tyr Asp Gly Ala Leu Lys Lys Gly Val Trp Asn Asp Met
340 345 350

Gly Thr Tyr Asn Val Asp His Leu Glu Ile Ile Gly Val Asp Pro Asn
355 360 365

Pro Ser Phe Asp Ile Arg Ala Phe Tyr Leu Arg Leu Ala Glu Gln Leu
370 375 380

Ala Ser Leu Arg Pro
385

<210> 6
<211> 417
<212> PRT
<213> Geobacillus thermoleovorans

<400> 6

```
Met Met Lys Gly Cys Arg Val Met Val Val Leu Leu Gly Leu Trp Phe
1               5                   10                  15

Val Phe Gly Leu Ser Val Pro Gly Gly Arg Thr Glu Ala Ala Ser Pro
            20                  25                  30

Arg Ala Asn Asp Ala Pro Ile Val Leu Leu His Gly Phe Thr Gly Trp
        35                  40                  45

Gly Arg Glu Glu Met Leu Gly Phe Lys Tyr Trp Gly Gly Val Arg Gly
    50                  55                  60

Asp Ile Glu Gln Trp Leu Asn Asp Asn Gly Tyr Arg Thr Tyr Thr Leu
65                  70                  75                  80

Ala Val Gly Pro Leu Ser Ser Asn Trp Asp Arg Ala Cys Glu Ala Tyr
                85                  90                  95
```

Ala Gln Leu Val Gly Gly Thr Val Asp Tyr Gly Ala Ala His Ala Ala
100 105 110

Asn Asp Gly His Ala Arg Phe Gly Arg Thr Tyr Pro Gly Leu Leu Pro
115 120 125

Glu Leu Lys Arg Gly Gly Arg Val His Ile Ile Ala His Ser Gln Gly
130 135 140

Gly Gln Thr Ala Arg Met Leu Val Ser Leu Leu Glu Asn Gly Ser Gln
145 150 155 160

Glu Glu Arg Glu Tyr Ala Lys Glu His Asn Val Ser Leu Ser Pro Leu
165 170 175

Phe Glu Gly Gly His Arg Phe Val Leu Ser Val Thr Thr Ile Ala Thr
180 185 190

Pro His Asp Gly Thr Thr Leu Val Asn Met Val Asp Phe Thr Asp Arg
195 200 205

Phe Phe Asp Leu Gln Lys Ala Val Leu Glu Ala Ala Ala Val Ala Ser
210 215 220

Asn Ala Pro Tyr Thr Ser Glu Ile Tyr Asp Phe Lys Leu Asp Gln Trp
225 230 235 240

Gly Leu Arg Arg Glu Pro Gly Glu Ser Phe Asp His Tyr Phe Glu Arg
245 250 255

Leu Lys Arg Ser Pro Val Trp Thr Ser Thr Asp Thr Ala Arg Tyr Asp
260 265 270

Leu Ser Val Pro Gly Ala Glu Thr Leu Asn Arg Trp Val Lys Ala Ser
275 280 285

Pro Asn Thr Tyr Tyr Leu Ser Phe Ser Thr Glu Arg Thr Tyr Arg Gly
290 295 300

Ala Leu Thr Gly Asn Tyr Tyr Pro Glu Leu Gly Met Asn Ala Phe Ser
305 310 315 320

Ala Ile Val Cys Ala Pro Phe Leu Gly Ser Tyr Arg Asn Ala Ala Leu
325 330 335

Gly Ile Asp Ser His Trp Leu Gly Asn Asp Gly Ile Val Asn Thr Ile
340 345 350

Ser Met Asn Gly Pro Lys Arg Gly Ser Asn Asp Arg Ile Val Pro Tyr
355 360 365

Asp Gly Thr Leu Lys Lys Gly Val Trp Asn Asp Met Gly Thr Tyr Lys
370 375 380

Val Asp His Leu Glu Val Ile Gly Val Asp Pro Asn Pro Ser Phe Asn
385 390 395 400

Ile Arg Ala Phe Tyr Leu Arg Leu Ala Glu Gln Leu Ala Ser Leu Arg
405 410 415

Pro

<210> 7
<211> 416
<212> PRT
<213> Geobacillus thermoleovorans

<400> 7

Met Lys Cys Cys Arg Val Met Phe Val Leu Leu Gly Leu Trp Leu Val
1               5                   10                  15

Phe Gly Leu Ser Val Pro Gly Gly Arg Ala Glu Ala Ala Thr Ser Arg
            20                  25                  30

Ala Asn Asp Ala Pro Ile Val Leu Leu His Gly Phe Thr Gly Trp Gly
        35                  40                  45

Arg Glu Glu Met Phe Gly Phe Lys Tyr Trp Gly Gly Val Arg Gly Asp
    50                  55                  60

Ile Glu Gln Trp Leu Asn Asp Asn Gly Tyr Arg Thr Tyr Thr Leu Ala
65                  70                  75                  80

Val Gly Pro Leu Ser Ser Asn Trp Asp Arg Ala Cys Glu Ala Tyr Ala
85 90 95

Gln Leu Val Gly Gly Thr Val Asp Tyr Gly Ala Ala His Ala Ala Lys
100 105 110

His Gly His Ala Arg Phe Gly Arg Thr Tyr Pro Gly Leu Leu Pro Glu
115 120 125

Leu Lys Arg Gly Gly Arg Ile His Ile Ile Ala His Ser Gln Gly Gly
130 135 140

Gln Thr Ala Arg Met Leu Val Ser Leu Leu Glu Asn Gly Ser Gln Glu
145 150 155 160

Glu Arg Glu Tyr Ala Lys Ala His Asn Val Ser Leu Ser Pro Leu Phe
165 170 175

Glu Gly Gly His His Phe Val Leu Ser Val Thr Thr Ile Ala Thr Pro
180 185 190

His Asp Gly Thr Thr Leu Val Asn Met Val Asp Phe Thr Asp Arg Phe
195 200 205

Phe Asp Leu Gln Lys Ala Val Leu Glu Ala Ala Ala Val Ala Ser Asn
210 215 220

Ala Pro Tyr Thr Ser Glu Ile Tyr Asp Phe Lys Leu Asp Gln Trp Gly
225 230 235 240

Leu Arg Arg Glu Pro Gly Glu Ser Phe Asp His Tyr Phe Glu Arg Leu
245 250 255

Lys Arg Ser Pro Val Trp Thr Ser Thr Asp Thr Ala Arg Tyr Asp Leu
260 265 270

Ser Val Pro Gly Ala Glu Thr Leu Asn Arg Trp Val Lys Ala Ser Pro
275 280 285

Asn Thr Tyr Tyr Leu Ser Phe Ser Thr Glu Arg Thr Tyr Arg Gly Ala
290 295 300

Leu Thr Gly Asn Tyr Tyr Pro Glu Phe Gly Met Asn Ala Phe Ser Ala
305 310 315 320

Ile Val Cys Ala Pro Phe Phe Gly Ser Tyr Arg Asn Ala Ala Leu Gly
325 330 335

Ile Asp Ser His Trp Leu Glu Asn Asp Gly Ile Val Asn Thr Ile Ser
340 345 350

Met Asn Gly Pro Lys Arg Gly Ser Ser Asp Arg Ile Val Pro Tyr Asp
355 360 365

Gly Ala Leu Lys Lys Gly Val Trp Asn Asp Met Gly Thr Tyr Asn Val
370 375 380

Asp His Leu Glu Ile Ile Gly Val Asp Pro Asn Pro Ser Phe Asp Ile
385 390 395 400

Arg Ala Phe Tyr Leu Arg Leu Ala Glu Gln Leu Ala Ser Leu Arg Pro
405 410 415

<210> 8
<211> 418
<212> PRT
<213> Bacillus sp. TP10A.1

<400> 8

Met Met Lys Cys Cys Arg Arg Val Ala Leu Val Leu Leu Gly Leu Trp
1 5 10 15

Leu Val Phe Cys Leu Ser Val Pro Gly Gly Arg Ala Glu Ala Ala Ala
20 25 30

Ser Arg Ala Asn Asp Ala Pro Ile Val Leu Leu His Gly Phe Thr Gly
35 40 45

Trp Gly Arg Glu Glu Met Phe Gly Phe Lys Tyr Trp Gly Gly Val Arg
50 55 60

Gly Asp Ile Glu Gln Leu Leu Asn Ala Gln Gly Tyr Arg Thr Tyr Thr
65 70 75 80

Leu Ala Val Gly Pro Leu Ser Ser Asn Trp Asp Arg Ala Cys Glu Ala
85 90 95

Tyr Ala Gln Leu Val Gly Gly Thr Val Asp Tyr Gly Ala Ala His Ala
100 105 110

Ala Lys His Gly His Ala Arg Phe Gly Arg Thr Tyr Pro Gly Leu Leu
115 120 125

Pro Glu Leu Lys Arg Gly Gly Arg Val His Ile Ile Ala His Ser Gln
130 135 140

Gly Gly Gln Thr Ala Arg Met Leu Val Ser Leu Leu Glu Asn Gly Ser
145 150 155 160

Lys Glu Glu Arg Glu Tyr Ala Lys Ala His Asn Val Ser Leu Ser Pro
165 170 175

Leu Phe Glu Gly Gly His Asn Phe Val Leu Ser Val Thr Thr Ile Ala
180 185 190

Thr Pro His Asp Gly Thr Thr Leu Val Asn Met Val Asp Phe Thr Asp
195 200 205

Arg Phe Phe Asp Leu Gln Lys Ala Val Leu Lys Ala Ala Ala Val Ala
210 215 220

Ser Asn Val Pro Tyr Thr Ser Gln Val Tyr Asp Phe Lys Leu Asp Gln
225 230 235 240

Trp Gly Leu Arg Arg Gln Pro Gly Glu Ser Phe Asp Gln Tyr Phe Glu
245 250 255

Arg Leu Lys Gln Ser Pro Val Trp Thr Ser Ala Asp Thr Ala Arg Tyr
260 265 270

Asp Leu Ser Val Pro Gly Ala Glu Ala Leu Asn Gln Trp Val Gln Ala
275 280 285

Ser Pro Asn Thr Tyr Tyr Leu Ser Phe Ala Thr Glu Arg Thr Tyr Arg
290 295 300

Gly Ala Leu Thr Gly Asn Tyr Tyr Pro Glu Leu Gly Met Asn Val Phe
305 310 315 320

Ser Ala Ala Val Cys Ala Pro Phe Leu Gly Ser Tyr Arg Asn Ala Ala
325 330 335

Leu Gly Ile Asp Asp Arg Trp Leu Glu Asn Asp Gly Ile Val Asn Thr
340 345 350

Phe Ser Met Asn Gly Pro Lys Arg Gly Ser Thr Asp Arg Ile Val Pro
355 360 365

Tyr Asp Gly Thr Leu Lys Lys Gly Val Trp Asn Asp Met Gly Thr Tyr
370 375 380

Asn Val Asp His Leu Glu Val Ile Gly Val Asp Pro Asn Pro Leu Phe
385 390 395 400

Asp Ile His Ala Phe Tyr Leu Arg Leu Ala Glu Gln Leu Ala Ser Leu
405 410 415

Arg Pro

<210> 9
<211> 20
<212> DNA
<213> primer sequence

<400> 9
agrgtttgat cmtggctcag

<210> 10
<211> 18
<212> DNA
<213> primer sequence

<400> 10
gktaccttgt tacgactt

<210> 11
<211> 20
<212> DNA
<213> primer sequence

<400> 11
taatacgact cactataggg 20

<210> 12
<211> 19
<212> DNA
<213> primer sequence

<400> 12
gctagttatt gctcagcgg 19

<210> 13
<211> 1152
<212> DNA
<213> Bacillus OK1

<400> 13

```
atgcgccgat tgtacttctc catggcttta ctggctgggg aagagaagaa atgtttgggt     60
tcaagtactg gggcggcgtg cgcggcgata tcgaacaatg gctgaacgac aacggttatc    120
gaacttatac gctggcggtc ggaccgctct cgagcaactg ggaccgggcg tgtgaagcgt    180
atgctcagct tgtcggcggg acggtcgatt atggggcagc ccatgcggcg aagcacggcc    240
atgcgcggtt tggccgcact tatcccggcc tgttgccgga attgaaaagg ggtggccgca    300
tccatatcat cgcccacagc caaggggggc agacggcccg catgcttgtc tcgctcctag    360
agaacggaag ccaagaagag cgggagtacg ccaaggcgca caacgtgtcg ttgtcaccgt    420
tgtttgaagg tggacatcat gttgtgttga gtgtgacgac catcgccact cctcatgacg    480
ggacgacgct tgtcaacatg gttgatttca ccgatcgctt ttttgacttg caaaaagcgg    540
tgttggaagc ggcggctgtc gccagcaacg tgccgtacac gagtcaagta tacgatttta    600
agctcgacca atggggactg cgccgccagc cgggtgaatc gttcgaccat tattttgaac    660
ggctcaagcg ctcccctgtt tggacgtcga cagataccgc ccgctacgat ttatccgttt    720
ccggagctga gaagttgaat caatgggtgc aagcaagccc gaatacgtat tatttgagct    780
ttgccacaga acggacgtat cgcggagcgc tcacaggcaa ctattatccc gaactcggaa    840
tgaatgcatt cagcgcggtc gtatgcgctc cgtttctcgg ttcgtaccgc aatccgacgc    900
tcggcattga cgaccgctgg cttgaaaacg atggcattgt caatacggtt tccatgaacg    960
gtccaaagcg tggatcaagt gatcggatcg taccgtatga cggggcgttg aaaaaagggg   1020
tttggaatga catgggaacg tacaatgtcg accatttgga aatcatcggc gttgacccga   1080
atccgtcatt tgatattcgc gccttttatt tgcgacttgc cgagcagttg gcgagtttgc   1140
ggccttaagc tt                                                       1152
```

<210> 14
<211> 1156
<212> DNA
<213> Bacillus F4b

<400> 14

```
atgcacccat cgtgcttctc catggtttta ccggctgggg aagagaagaa atgtttgggt   60
tcaagtactg gggcggcgtg cgcggcgata tcgaacaatg gctgaacgac aacggttatc  120
gaacttatac gctggcggtc ggaccgctct cgagcaactg ggaccgggcg tgtgaagcgt  180
atgctcagct tgtcggcggg acggtcgatt atggggcagc ccatgcggca aagcacggcc  240
atgcgcggtt tggccgcacg tatccggggt tgctgccgga attgaaaaga ggaggccgcg  300
tccatatcat cgcccacagc caaggagggc agacggcccg tatgcttgtc tcgctcctag  360
agaacggaag ccaagaagag cgggagtacg ccaaggagca caacgtgtcg ttgtcgccgt  420
tgtttgaagg cggacatcgt tttgtgttga gcgtgacgac catcgccact cctcatgacg  480
ggacgacgct tgtcaacatg gttgatttca ccgatcgctt ttttgacttg caaaaagcgg  540
tgttggaagc ggcggctgtc gccagcaacg tgccgtacac gagtcaagta tacgatttta  600
agctcgacca atggggactg cgccgccagc cgggtgaatc gttcgaccat tattttgaac  660
ggctcaagcg ctcccctgtc tggacatcga cagataccgc ccgctacgat ttatccgttt  720
ccggagctga gaagttgaat caatgggtgc aagcaagccc gaatacgtat tatttgagct  780
tttctaccga acggacgtat cgcggagcgc tcacaggcaa ctattatccc gaactcggaa  840
tgaatgcatt cagcgcggtc gtatgcgctc cgtttctcgg ttcgtaccgc aatccgacgc  900
tcggcattga cgaccgctgg cttgaaaacg atggtattgt caatacggtt tccatgaacg  960
gtccaaagcg tggatcaagc gatcggatcg taccgtatga cggggcgttg aaaaaagggg 1020
tttggaatga catgggaacg tacaatgtcg accatttgga aatcatcggc gttgacccga 1080
atccgtcatt tgatattcgc gccttttatt tgcggcttgc cgagcaactg gcgagtttgc 1140
ggccttaagc ttaagg                                                 1156
```

<210> 15
<211> 1152
<212> DNA
<213> Bacillus H2

<400> 15

```
atgcacccat cgtgcttctc catggtttta ccggctgggg aagagaagaa atgtttgggt    60
tcaagtactg gggcggcgtg cgcggcgata tcgaacaatg gctgaacgac aacggttatc   120
gaacttatac gctggcggtc ggaccgctct cgagcaactg ggaccgggcg tgtgaagcgt   180
atgctcagct tgtcggcggg acggtcgatt atggggcagc ccatgcggca aagcacggcc   240
atgcgcggtt tggccgcacg tatccggggt tgctgccgga attgaaaaga ggaggccgcg   300
tccatatcat cgcccacagc caaggagggc agacggcccg tatgcttgtc tcgctcctag   360
agaacggaag ccaagaagag cgggagtacg ccaaggagca caacgtgtcg ttgtcgccgt   420
tgtttgaagg cggacatcgt tttgtgttga gcgtgacgac catcgccact cctcatgacg   480
ggacgacgct tgtcaacatg gttgatttca ccgatcgctt ttttgacttg caaaaagcgg   540
tgttggaagc ggcggctgtc gccagcaacg tgccgtacac gagtcaagta tacgatttta   600
agctcgacca atggggactg cgccgccagc cgggtgaatc gttcgaccat tattttgaac   660
ggctcaagcg ctcccctgtc tggacatcga cagataccgc ccgctacgat ttatccgttt   720
ccggagctga gaagttgaat caatgggtgc aagcaagccc gaatacgtat tatttgagct   780
tttctaccga acggacgtat cgcggagcgc tcacaggcaa ctattatccc gaactcggaa   840
tgaatgcatt cagcgcggtc gtatgcgctc cgtttctcgg ttcgtaccgc aatccgacgc   900
tcggcattga cgaccgctgg cttgaaaacg atggtattgt caatacggtt tccatgaacg   960
gtccaaagcg tggatcaagc gatcggatcg tcccgtatga cggggcgttg aaaaaagggg  1020
tttggaatga catgggaacg tacaatgtcg accatttgga aatcatcggc gttgacccga  1080
atccgtcatt tgatattcgc gccttttatt tgcggcttgc cgagcaactg gcgagtttgc  1140
ggccttaagc tt                                                       1152
```

## Description

### FIELD OF INVENTION

This invention relates to the field of chemical production. In particular, though not exclusively, the invention relates to the field of biodiesel production.

### BACKGROUND

Biodiesel is a renewable biofuel, defined as fatty acid monoalkyl ester (FAME), formed from a refined mixture of alkyl esters which are usually, but not exclusively, methyl and ethyl esters of saturated and unsaturated long chain fatty acids derived from vegetable or microalgal oil or animal fat. Biodiesel can be used on its own, or mixed with conventional diesel fuel, in diesel (compression-ignition) engines. Biodiesel refers to the pure fuel which can be used in its pure form or blended with petroleum diesel.

Biodiesel is currently produced by the transesterification (or alcoholysis) of oils (reviewed in Fukuda, H et al J. Bioscience & Bioengineering, 2001, 92, 5, 405-416). Germany and France are the leading producers ofbiodiesel, but production in other countries, notably the United States, is increasing.

The current preferred commercial process for biodiesel production involves the transesterification of triglycerides in various oils, typically sunflower and rapeseed oil. Other seed oils such as mustard, soybean, sunflower, macadamia, coconut and peanut are also contemplated as a basis for biodiesel production. This chemical process, which is illustrated in the accompanying Figure 1, consists of a number of consecutive reversible reactions (shown in Figure 1B) and involves the reaction of the oil or fat, in this case triglyceride, with an alcohol, typically methanol, ethanol, propanol, butanol, or amyl alchol in the presence of an alkali catalyst, typically sodium methoxide, potassium hydroxide or sodium hydroxide, operating at or about 65°C. In this process, the fatty acids are removed from the glyceride molecule to produce, in this case, three fatty acid monoalkyl esters and glycerol ("glycerine" in US publications). The process often involves neutralisation with acetic acid or mineral acid resulting in a large and undesirable effluent stream. Glycerol is produced as a valuable by-product of the conventional process but it is contaminated and therefore has a lower value (about £150/tonne). The monoalkyl ester/alcohol mixture produced is separated and excess alcohol recycled. The monoalkyl esters require a purification process before use. The monoalkyl esters are usually purified by fractional distillation under a vacuum. An example of a conventional chemical process is given in United States patent application number 20030032826.

Biodiesel can also be produced by simultaneous catalytic hydrogenation and cracking of vegetable and tree oils and has been produced using an oil by-product from the Kraft pulping process.

Enzymes, in particular lipases, provide an alternative catalyst for this reaction. Lipases are a diverse class of enzymes that will catalyse the aqueous phase breakdown of fats or oils into fatty acids and glycerol. Alternatively triglycerides and partial glycerides may be transesterified by lipases to partial glycerides and free fatty acid esters in an essentially water free environment but in the presence of a suitable alcohol. This type of reaction is reversible, as shown in the accompanying Figure 1B.

In biodiesel production, lipases can be used to transesterify triglyceride with methanol, producing the desired fatty acid monoalkyl esters according to the reaction shown in Figure 1. By way of example, Abigor R D et al. (Biochemical Society Transactions (2000) 28 979-981) discloses lipase-catalysed production of biodiesel fuel from some lauric oils.

Du W, et al. (Biotechnol. Appl. Biochem. (2003) 38 103-6) discloses lipase-catalysed transesterification of soya bean oil for biodiesel production in continuous and non-continuous batch operation using the enzyme Lipozyme TL IM. Whilst in non-continuous batch operation, the optimal oil/alcohol ratio and temperature were 1:4 and 40-50°C; however, during the continuous batch operation, the optimal oil/alcohol ratio and temperature were 1:1 and 30°C; Hsu An C et al. (Biotechnology and Applied Biochemistry, (2002) 36 181-186) discloses lipase-catalyzed production of alkyl esters of restaurant grease, for use as biodiesel.

US-A-5,713,965 relates to the use of lipases to produce alkyl esters. Reactions were carried out using a 3:1 molar ratio mixture of alcohol and tallow over a temperature range of 35-55°C. The lipases used were derived from *Mucor miehei, Candida antarctica, Geotrichum candidum, Pseudomonas cepacia* and *Rhizopus delemar.* No *Bacillus* species were used.

US-A-5,935,828 relates to the production of ω-3 fatty acids using a lipase, comprising a step of transesterification of triglycerides in an alcohol medium. A reaction temperature in the range of 20-60°C is mentioned, although the example only discloses the use of 40°C. The lipase used is derived from *Pseudomonas fluorescens*.

WO-A-90/04033 relates to the production of monoglycerides by lipase-catalysed transesterification by combining oils or pure triglycerides with alcohol and a small amount of water in the presence of a lipase. A reaction temperature of 20-60°C, preferably 25-45°C, is disclosed, although the examples describe only 20°C or 45°C as the reaction temperature. The lipase used is derived from *Pseudomonas fluorescens*.

EP-A-0709465 relates to the production of optically active alcohol by an interesterification between a racemic alcohol and an ester in the presence of a lipase. US-A1-2005/0204612 relates to the production of biodiesel by esterification of free fatty acids with a monoalcohol in the presence of an acid catalyst, followed by two or more transesterification steps in the presence of a base catalyst. These documents do not relate to the production of biodiesel by transesterifying a triglyceride in an oil or fat feedstock with an alcohol in the presence of a lipase.

WPI abstract accession no. 1994-174015 is an abstract of JP6116585. The abstract relates to obtaining fats and oils having a large amount of docosahexaenoic acid (DHA) by reacting fats and oils containing DHA with alcohol in the presence of lipase and separating the resulting ester. A reaction temperature of 30°C is disclosed.

Extracellular and intracellular lipases have been used, the latter as whole cell biocatalysts (Brockerhoff & Jenson (eds) Lipolytic Enzymes, Academic Press (New York, 1974) p1-340; Borgstrom & Brockman (eds) Lipases, Elsevier (New York, 1984) p1-420) Disadvantages with current biodiesel production processes using lipases, as described in the above publications, include the high costs of enzymes, low catalytic productivity and the fact that the operational temperature of the lipase catalysed reactions is well below thermodynamic optimum for the reaction. It is likely that such low temperatures were used as the result of the optimum growth temperature of the organisms from which the lipases were derived (approximately 25°C or 37°C), such that the lipases are not stable at high temperatures (greater than 40°C). However, the transesterification reactions work faster at temperatures greater than 40°C, with an additional benefit that the viscosity of the feedstock is reduced, allowing it to be handled more easily. A further disadvantage is that the activity of mesophilic lipases is relatively low, for example as described in Watanabe et al. (JAOCS (2000) 77 355-360) in which the lipase reaction took 36h to complete, compared to 1h for conventional catalysts. The cost of lipases is very high compared to conventional strong alkali catalysts such as sodium methoxide, but has the benefit that it does not involve the creation of an effluent stream as the result of neutralisation with acetic acid or mineral acid. In addition, the use of lipases allows more specific hydrolysis, since lipase will only catalyse the transesterification of fatty acids. Base hydrolysis is less specific than an enzyme catalysed hydrolysis and there is potential for side reactions to occur.

JP1010994 (Kao Corporation) discusses the use of lipases in a different experimental system, namely, a system having a relatively high water:alcohol ratio, in the range 0.8-5 moles water: 1 mole alcohol. The molar ratio of alcohol: oil was 3:1. Lipases derived from *Pseudomonas, Rhisopus* and *Humicola* species of thermophilic bacteria are indicated to be suitable in such a method, with specific examples disclosed in relation to a particular strain of *Pseudomonas,* namely, *Pseudomonas* SP.KSM-16. The inventors of application JP1010994 were apparently able to produce fatty acid esters by transesterification in a system which was not free of water, contrary to the accepted trend in the prior art that the presence of water would result in the production of free fatty acids. They note that a water:alcohol ratio of less than 0.8:1 results in insufficient activation of the lipase, whilst a ratio of more than 5:1 results in a large amount of fatty acid being produced, with ester synthesis reduced. There is no mention of the purity of glycerol produced using this process.

Shimada et al (JAOCS 76 (1999) 789-793) relates to conversion of vegetable oil to biodiesel using a *Candida antarctica* lipase. Methanolysis experiments were carried out over the temperature range of 20-60°C for up to 24 hours. When more than 1.5 molar equivalents of methanol were present in the oil mixture, the rate of methanolysis was decreased. When the lipase was returned to a 1:1 oil/methanol mixture, lipase activity was not restored, indicating that the presence of methanol irreversibly inactivates this lipase. The paper notes that 3:1 methanol/oil equivalents are required for the complete conversion of oil to its corresponding methyl ester. The solution provided by the authors was to conduct a three step experiment, with 1:1 methanol/oil for 24 hours, addition of a second molar equivalent of methanol for a further 24 hours and addition of a further molar equivalent for a final 24 hour period. Using this three step process, conversion of oil to methyl ester of 98.4% was achieved. However, when using just the first, single step, 1:1 reaction, conversion of only around 32.5% was achieved. Increasing the reaction temperature did not improve this reaction.

Tan et al (App. Biochem. Biotechnol. 128 (2006) 109-116) discusses biodiesel production in an experimental system having high water content and using a *Candida* lipase. As with the method described by Shimada *et al,* methanol was added to the reaction mixture in three separate steps. The authors mention that methanol denatures lipase and indicate their view that methanol must, therefore, be added stepwise in order to reduce this denaturing effect.

Hirata et al (J. Biotechnol. 14 (1990) 157-167) is a review of the properties of lipases from *Candida cylindracea, Pseudomonas fluorescens, Penicillium cycolprium* and *Penicillium roqueforti*. This paper suggests the use of the presence of water to accelerate the rate of transesterification of triglycerides. The molar ratio of tributyrin:1-octanol used in the experiments was approximately 1:1.

WO01/53511 relates to esterification of sterols with carboxylic acids using *Candida antarctica* lipase A. There is no mention of transesterification of a triglyceride.

Basri et al (JAOCS 74 (1997) 113-116) relates to alcoholysis of palm oil mid fraction by *Rhizopus rhizopodiformis*. Reactions were carried out at 37°C with the optimal oil:methanol molar ratio being indicated to be 1:1 - 1:2. The lipase was shown to retain activity at 70°C for around 22 hours.

Goma-Donescu & Legoy (JAOCS 74 (1997) 1137-1143) relates to transesterification of vegetable oils by a commercial lipase obtained from *Mucor miehei.* Reactions were carried out at between 55°C and 65°C.

Li & Zhang (Protein Expression & Purification 42 (2005) 153-159) relates to characterisation of a lipase from *Geobacillus* sp. *TW1.* Lipase activity was determined for the temperature range of 10-90°C for 15 minutes. The effect of pre-incubation of the lipase for 15 minutes over the temperature range 40-90°C on subsequent residual activity at 60°C was also determined. The lipase was found to retain activity and to remain thermostable to some extent over these temperature ranges, although no test of activity or stability for a period of time longer than 15 minutes was reported.

US2006/0024789A1 relates to characterisation of a lipase from *Geobacillus* strain T1. The lipase was shown to retain activity for 30 minutes at up to 80°C. There is no indication of measurement of activity for longer periods of time. EP1624056 relates to the same subject matter, both applications claiming priority from MY0403110.

Abdel-Fattah & Gaballa (Microbiological Research (2006) Article in Press) relates to the identification of a lipase from *Geobacillus thermoleovorans* Toshki, in seeking to promote catalysis of enantiospecific hydrolysis reactions for fine chemical production. Lipase activity was tested after 1 hour of incubation at temperatures of up to 100°C. There is no indication of the longer term stability of the enzyme at elevated temperatures and no discussion ofbiodiesel production.

Tyndall et al (J. Mol. Biol. 323 (2002) 859-869) is a discussion of the crystal structure of a lipase from *Bacillus stearothermophilus* P1, including a discussion of factors which may contribute to protein thermostability.

Cho et al (FEMS 186 (2000) 235-238) relates to a lipase from *Bacillus thermoleovorans* ID-1. Lipase activity was determined after exposure to various temperatures for 1 hour, with no loss of activity observed after incubation at 50°C for several hours. There is no discussion of any longer term stability of the enzyme at more elevated temperatures.

Sunna et al (Enzyme & Microbial Technology 31 (2002) 472-476) is a description of the characterisation of a lipase derived from *Bacillus* sp. Tp10A.1, in seeking to promote catalysis of enantiospecific hydrolysis reactions for fine chemical production. Hydrolysis activity of the lipase is disclosed, with activity being shown at up to 80°C when the lipase was incubated at this temperature for 5 minutes. There is no disclosure of transesterification activity or of the activity or stability of the enzyme at high temperatures for longer periods of time and no discussion of biodiesel production.

It is an object of the present invention to provide improved lipases and methods of producing biodiesel and/or glycerol. In particular, it is an object of the invention to provide a biodiesel production process which can be operated at higher temperatures than conventional processes having a low water content, preferably operating at more than 65°C, the upper limit of stability for mesophilic lipases. In this case the enzyme will be functionally active at high temperatures, with consequent advantages such as an increase in reaction rate and reduction in viscosity of a vegetable oil substrate.

## SUMMARY OF THE INVENTION

According to a first aspect of the invention, there is provided an isolated lipase expressed by a *Bacillus* cell which has detectable lipase activity at a temperature of 80°C for a period of at least 1,5 hours, wherein the lipase is not expressed by *Bacillus* sp. Tp10A.1, and wherein the lipase is produced by any one of *Bacillus* strains NCIMB41255 (N2b), NCIMB41256 (OK1) and NCIMB41257 (N13a) and comprises the amino acid sequence of SEQ ID NO:3.

According to a second aspect of the invention there is provided an isolated lipase expressed by a *Bacillus* cell, wherein the lipase has detectable lipase activity at a temperature of 80°C for a period of at least 1.5 hours, wherein the lipase is not expressed by *Bacillus* sp. Tp10A.1, and wherein the lipase is produced by any one of *Bacillus* strains NCIMB41254 (F4b), NCIMB41258 (G6) and NCIMB41259 (H2), and wherein the lipase has 98.5% sequence homology to SEQ ID NO:4.

Preferably, the lipase activity is transesterification activity. Advantageously, such a lipase may be used to catalyse a transesterification reaction to produce biodiesel at a more optimal temperature for the reaction than in methods disclosed in the prior art, enabling industrial scale use of a lipase-catalysed biodiesel production process. A lipase according to the invention remains active at such a temperature for a considerable period of time and, indeed, remains active for several hours even at more elevated temperatures.

Preferably, the lipase has detectable lipase activity at a temperature of 80°C for a period of at least 3.5 hours and more preferably for a period of at least 5.5 hours. Yet more preferably, the lipase has detectable lipase activity at a temperature of 82°C for a period of at least 2 hours and most preferably for a period of at least 2.5 hours.

The term "detectable" indicates that the activity of the lipase is measurable and can be measured using, for example, methods set out in the present specification. For example, lipase activity can be measured by detection of fatty acid monoalkyl ester (FAME) production, or by detection of tributyrin hydrolysis.

Preferred lipases include those produced by strains H2, H3, G6, F1, F3, F4b, F12, N2a, N2b, N13a, N13b, WM3, OK1, OK2, HG3a, a selection of which strains have been deposited at the National Collection of Industrial, Marine and Food Bacteria (NCIMB), Ferguson Building, Craibstone Estate, Bucksburn, Aberdeen, AB21 9YA, United Kingdom, in accordance with the Budapest Treaty, under the accession numbers NCIMB41254 (F4b), NCIMB41255 (N2b), NCIMB41256 (OK1), NCIMB41257 (N13a), NCIMB41258 (G6) and NCIMB412S9 (H2).

In the first aspect of the invention the lipase is produced by any one of *Bacillus* strains NCIMB41255 (N2b), NCIMB41256 (OK1) and NCIMB41257 (N13a) and comprises the amino acid sequence of SEQ ID NO:3.

In the second aspect of the invention, the lipase is produced by any one of *Bacillus* strains NCIMB412S4 (F4b), NCIMB41258 (G6) and NCIMB41259 (H2) and has at least 98.5% sequence homlogy to SEQ ID NO:4. The lipase may comprise the amino acid sequence of SEQ ID NO:4.

Preferably, the lipase has at least 98.5% sequence identity to SEQ ID NO:4. More preferably, the lipase may comprise an amino acid sequence having at least 99% or at least 99.5% sequence homology to SEQ ID NO:4. Preferably, the lipase has at least 99% or at least 99.5% sequence identity to SEQ ID NO:4. The lipase may be non-naturally occurring.

The lipase may be encoded by a polynucleotide sequence having at least 97.5% sequence homology to any one of SEQ ID NO:13, SEQ ID NO:14 or SEQ ID NO:15. Preferably, the lipase is encoded by a polynucleotide sequence having at least 97.5%, 98% or 99% sequence identity to any one of SEQ ID NO:13, SEQ ID NO:14 or SEQ ID NO:15.

The term "homology", as used throughout this specification, is intended to indicate the level of functional and/or structural similarity between polypeptide sequences (or, in the case of polynucleotide sequences, the polypeptide sequence resulting from the expression thereof), allowing for both the degeneracy of the genetic code and the chemical and functional similarity between certain amino acids (e.g. polar amino acids, charged amino acids and hydrophobic amino acids). Functional similarity indicates that the polynucleotides or polypeptides being compared have substantially identical functionality, i.e. that the polypeptide is, or the polynucleotide encodes, a lipase expressed by a *Bacillus* cell which has detectable lipase activity at a temperature of 80°C for a period of at least 1.5 hours.

The term "sequence identity", as used throughout this specification, is calculated as the percentage of nucleotides or amino acids present in the smaller of the two sequences to be compared that may also be found in the larger of the two sequences, the nucleotides or amino acids preferably being arranged in the same order in both sequences.

The skilled person would readily be able to determine the level of sequence homology or identity between sequences, for example by use of the Blast analysis tool at http://www.ncbi.nln.nih.gov/BLAST/, using the default parameter settings.

Preferably, the length of the shorter of the two sequences being compared is at least 60% of the length of the longer of the two sequences, more preferably at least 70% of the length, yet more preferably at least 80% of the length and still more preferably at least 90%, 95%, 96% 97% 98% or 99% of the length. In a most preferred embodiment, the sequences to be compared are identical in length.

Described below is a method for the production of biodiesel comprising transesterifying a triglyceride in an oil or fat feedstock with an alcohol in the presence of a lipase isolated from a *Bacillus* species, wherein the initial molar ratio of triglyceride:alcohol is 1:3.

Advantageously, the use of such a method allows the production of biodiesel in a single-step reaction which can proceed to completion at industrially applicable temperatures.

The oil or fat feedstock may be of vegetable or animal origin. For example, the feedstock may be derived from vegetable or microalgal oil or animal fat. Where the feedstock is of vegetable origin it may typically be sunflower, palm and/or rapeseed oil. Oils from other seeds such as mustard, soybean, sunflower, macadamia, coconut and/or peanut are also contemplated for use in the methods of the invention as well as waste vegetable oils. Where the fat is of animal origin it may be tallow.

Typically, the alcohol will be methanol. Other alcohols such as ethanol, propanol, isopropanol or other short chain alcohols (i.e. alcohols having between 1 and 18 carbon atoms) can be used. Methanol is preferred as it is low cost and solvates well in current chemically catalysed processes. Other alcohols could be used to produce e.g. ethyl and higher esters, depending on the fuel value of the different esters and the economies of using a higher alcohol in the transesterification process.

Preferably, the lipase is expressed by a *Bacillus* cell which has detectable lipase activity at a temperature of 80°C for a period of at least 1.5 hours. In one embodiment, the lipase is not expressed by *Bacillus* sp. Tp10A.1.

Preferably, the lipase has detectable lipase activity at a temperature of 80°C for a period of at least 3.5 hours and more preferably for a period of at least 5.5 hours. Yet more preferably, the lipase has detectable lipase activity at a temperature of 82°C for a period of at least 2 hours and most preferably for a period of at least 2.5 hours.

Preferred lipases include those produced by strains H2, H3, G6, F1, F3, F4b, F12, N2a, N2b, N13a, N13b, WM3, OK1, OK2, HG3a.

More preferably, the lipase is produced by any one of *Bacillus* strains NCIMB41255 (N2b), NCIMB41256 (OK1) and NCIMB41257 (N13a). The lipase may comprise the amino acid sequence of SEQ ID NO:3.

In an alternative preferred embodiment, the lipase is produced by any one of *Bacillus* strains NCIMB41254 (F4b), NCIMB41258 (G6) and NCIMB41259 (H2). The lipase may comprise the amino acid sequence of SEQ ID NO:4

The lipase may comprise an amino acid sequence having at least 98.5% sequence homology to SEQ ID NO: 4. Preferably, the lipase has at least 98.5% sequence identity to SEQ ID NO:4. More preferably, the lipase may comprise an amino acid sequence having at least 99% or at least 99.5% sequence homology to SEQ ID NO:4. Preferably, the lipase has at least 99% or at least 99.5% sequence identity to SEQ ID NO:4. The lipase may be non-naturally occurring.

The method may be carried out at a temperature of at least 55°C, preferably at least 60°C, more preferably at least 65°C. Alternatively or additionally, the method may be carried out in the absence of water.

Preferably, the lipase activity is not inhibited by the presence of the alcohol. Such inhibition can be determined, for example by measuring the rate of the transesterification reaction for extended periods of time. Were the lipase activity to be inhibited by the presence of alcohol, the rate of the reaction would be expected to be reduced.

Preferably, the lipase is in an immobilised form, in cells or as isolated enzyme. For example, the lipase may be immobilised on Celite® (Lake Chemicals & Minerals, Worcestershire, UK; also available from Fisher (Acros), Loughborough, UK), sepharose beads, glass beads, fibrous materials such as loofah, zeolites, resins and polymeric materials. Suitable supports are well known to the skilled person who would readily be able to determine how to immobilise the lipase (whether within cells or as isolated enzyme) on the support.

There may be provided the use of a lipase, which is expressed by a *Bacillus* cell and which has detectable lipase activity at a temperature of 80°C for a period of at least 1.5 hours, as a catalyst in a method comprising transesterifying a triglyceride in an oil or fat feedstock with an alcohol, wherein the initial molar ratio of triglyceride:alcohol is 1:3.

Advantageously, such a use allows the production of biodiesel in a single-step reaction which can proceed to completion at industrially applicable temperatures. The lipase remains active at such a temperature for a considerable period of time and, indeed, remains active for several hours even at more elevated temperatures.

In one embodiment, the lipase is not expressed by *Bacillus* sp. Tp10A.1. The lipase may be a lipase according to the first aspect of the invention.

According to a third aspect of the invention there are provided novel microorganisms deposited as NCIMB41254 (F4b), NCIMB41255 (N2b), NCIMB41256 (OK1), NCIMB41257 (N13a), NCIMB41258 (G6) or NCIMB41259 (H2).

According to a fourth aspect of the invention, there is provided a method of isolating a lipase expressed by a *Bacillus* cell which has detectable lipase activity at a temperature of 80°C for a period of at least 1.5 hours, wherein the lipase is not expressed by *Bacillus* sp. Tp10A.1, and wherein the lipase is produced by any one of *Bacillus* strains NCIMB41255 (N2b), NCIMB41256 (OK1) and NCIMB41257 (N13a) the method comprising:

a) determining whether the *Bacillus* cell is expressing a lipase;
b) determining whether said lipase has detectable lipase activity at a temperature of 80°C for a period of at least 1.5 hours; and
c) isolating said lipase,

wherein steps (b) and (c) may be carried out in any order.

The steps may be completed using, for example, methods set out in the present specification.

There may be provided a lipase obtainable or obtained using the method according to the fourth aspect of the invention. Preferably, the lipase has been isolated using the method according to the fourth aspect of the invention and, more preferably, is not expressed by *Bacillus* sp. Tp10A.1.

## BRIEF DESCRIPTION OF THE DRAWINGS

Figure 1 is taken from Fukuda, H *et al* 2001 *supra* and illustrates a general reaction for transesterification of triglyceride with alcohol: (A) General reaction, (B) three consecutive reactions R1, R2, R3 and R' represent alkyl groups.

Lipases and methods in accordance with the invention will be described, by way of example only, with reference to the further accompanying Figures 2-17 in which:

Figures 2A and 2B are graphs illustrating the separation of fatty acid monoalkyl esters (FAMEs) by gas chromatography: (2A) shows separation of standard FAMEs, (2B) shows separation of the products of chemical transmethylation of rapeseed oil;

Figure 3 is a graph illustrating the separation of the products of lipase-catalysed transmethylation at 40°C of rapeseed oil;

Figures 4A and 4B are graphs illustrating the separation of FAMEs by gas chromatography: (4A) shows separation of standard FAMEs, (4B) shows separation of products of F4b lipase-catalysed transmethylation at 60°C of rapeseed oil;

Figures 5, 6, 7 and 8 show alignments of amino acid sequences of various lipases;

Figure 9 shows lipase activity in cells at 80°C for 1.5 hours;

Figure 10 shows lipase activity in cells at 80°C for 2.5 hours;

Figure 11 shows lipase activity in cells at 80°C for 3.5 hours;

Figure 12 shows lipase activity in cells at 80°C for 5.5 hours;

Figure 13 shows lipase activity in cells at 82°C for 2 hours;

Figure 14 shows lipase activity in cells at 82°C for 2.5 hours;

Figure 15 shows lipase activity in cells at 82°C for 4 hours;

Figure 16 shows lipase activity in cells at 82°C for 3.5 hours; and

Figures 17A-D show the alignment of the DNA sequences found in various bacterial strains to encode various lipases.

## EXAMPLES

### Materials

LB media

| | |
|---|---|
| Liquid: | 10g tryptone<br>10g NaCl;<br>5g yeast extract.<br>made up to 1 litre with deionised water and autoclaved. |
| Solid: | As for liquid medium with the addition of 1.5% Phytagel™ (Sigma Aldrich, St Louis, Missouri, USA; product no. P8169) prior to autoclaving |

Minimal media

| | |
|---|---|
| Liquid: | 0.2g yeast extract<br>20ml rapeseed oil (2%)<br>made up to 1 litre with deionised water and autoclaved.<br><br>10% (v/v) Castenholz salts solution (see below) was added, and the medium was then homogenised to emulsify the oil. |
| Solid: | As for liquid minimal medium, but with 0.5% rapeseed oil.<br>2.5% Phytagel™ was added prior to autoclaving.<br>The decreased oil concentration and increased Phytagel™ concentration were required to increase to stability of the plates at 60°C. |

Castenholz salts solution

1.0g nitrilotriacetic acid
0.42g $CaSO_4 \cdot 2H_2O$
1.0g $MgSO_4 \cdot 7H_2O$
0.08g NaCl
1.03g $KNO_3$
6.89g $NaNO_3$
2.8g $Na_2HPO_4 \cdot 12H_2O$
10ml Nitsch's solution
10ml $FeCl_3$ solution (0.047g $FeCl_3 \cdot 6H_2O$ in 100ml)

made up to 1 litre with deionised water, and adjusted to pH 8.2 with 10M NaOH, then filter sterilised.

Nitsch's solution

0.5ml $H_2SO_4$
2.9g $MnSO_4 \cdot 4H_2O$
0.5g $ZnSO_4 \cdot 7H_2O$
0.5g $H_3BO_3$
0.025g $CuSO_4 \cdot 5H_2O$
0.025g $Na_2MoO_4 \cdot 2H_2O$
0.046g $CoCl_2 \cdot 6H_2O$

made up to 1 litre with deionised water and autoclaved.

**Identification of lipases**

Using a targeted strategy designed to isolate lipase producers, 12 new strains were isolated from environmental samples taken from several locations suitable for thermophilic organisms. Initial screening of these strains indicated that they possess varying levels of cell-associated and extracellular lipase activity.

*i) Thermophiles from CER culture collection*

14 strains were identified from the Centre for Extremophile Research, University of Bath (CER) culture collection as organisms growing at 60-70°C with growth temperature optima of 55-65°C. The strains had been isolated previously from samples collected in Montserrat. For further details of sampling sites and isolation procedures, see Atkinson et al (Extremophiles 4, 305, 2000). Liquid cultures were established initially on standard nutrient medium, prior to plating on selective media. Specifically, cryopreserved samples of each strain were used to inoculate LB plates, which were inverted, sealed in Sterilin bags and incubated at 60°C. Initially the plates were prepared with 1.5% Phytagel™, but this was subsequently increased to 2.5% in order to maintain the integrity of the solid medium at 60°C. A single colony from each plate was used to inoculate liquid LB medium; liquid cultures were incubated at 60°C without agitation and sub-cultured weekly.

Where necessary, bacterial growth was monitored by $OD_{600}$ measurements. A sample from each culture (1 ml) was removed and centrifuged at 16500 x g for 10 min to pellet the cells, which were then resuspended in 1 ml 50 mM phosphate buffer, pH 7.2 for measurement of $OD_{600}$. This eliminated problems arising from turbidity due to the presence of rapeseed oil in the growth medium. For growth on minimal medium plates, isolates were grown on minimal medium plates containing 2.5% Phytagel™ with 0.5% rapeseed oil as the sole carbon source. Plates were incubated at 60°C as above; colony growth and the presence of clearing zones in the turbid medium were recorded. Isolates showing growth with clearing zones were inoculated into liquid minimal medium containing rapeseed oil, incubated at 60°C and sub-cultured weekly. 11 of the 14 strains showed good growth on nutrient medium and so were selected for further investigation.

The 11 strains were further characterised by their ability to grow on a selective medium. Previous work had shown that certain thermophilic strains were able to grow on tributyrin (glyceryl tributyrate). In this work, the selective media used were:

minimal salts + 0.5% tributyrin
minimal salts + 0.5% rapeseed oil

Of the 11 strains, 4 showed growth on tributyrin while 10 showed growth on rapeseed oil. In most cases (7/10), growth on rapeseed oil was accompanied by the formation of a clearing zone surrounding bacterial colonies (Table 1). This indicates that the cells were secreting an extracellular lipase during growth on the lipid nutrient.

**Table 1. Growth of thermophilic strains on 0.5% rapeseed oil**

| Strain | Result after 7 days incubation | | |
|---|---|---|---|
| | Montserrat Sample site | Growth | Clearing |
| H2 | Hot Water Pond | + + | + + + |
| H3 | Hot Water Pond | + + | + + |
| G6 | Galway's Soufriére | + + | + + + |
| G8 | Galway's Soufriére | + | - |
| G11 | Galway's Soufriére | + | - |
| F1 | Hot Water Pond | ++ | +++ |
| F2 | Hot Water Pond | - | - |
| F3 | Hot Water Pond | + + | +++ |
| F4a | Hot Water Pond | + | - |
| F4b | Hot Water Pond | + + | + |
| F12 | Hot Water Pond | + | + + + |

*ii) Isolation of novel thermophilic lipase producers*

The thermophilic strains identified from the CER Culture Collection were originally isolated on non-selective media. In order to identify additional thermophilic, lipase-producing strains, novel organisms from appropriate environmental samples were isolated using a selective enrichment procedure.

12 environmental samples from the CER collection were identified as meeting criteria appropriate for this project. These samples had been collected from geothermally active locations in New Zealand and Italy at temperatures >80°C and at pH 2 - 7.5. In particular, soil samples were taken from geothermally active regions in Italy (Naples area) and New Zealand. A small loop of soil from each of the soil samples was added to minimal medium containing 2% rapeseed oil (5 ml) and incubated at 60°C with occasional shaking. When growth was noted, samples were sub-cultured by application of a 100μl sample at 1/10,000 dilution onto the surface of a minimal medium plate containing 2.5% Phytagel™ and 0.5% rapeseed oil. Plates were incubated for 7 days, single colonies then being used to reinoculate liquid minimal medium (5 ml) as above.

Samples taken through 5 rounds of subculture (1 round termed as being growth in liquid culture followed by growth on solid medium) were then designated as 'isolates' and given an appropriate code.

Lipase-producing strains were isolated by 5 rounds of selective enrichment, each round comprising:

- growth in liquid medium (minimal salts + 2% rapeseed oil) at 60°C;
- streaking onto solid medium (Phytagel™ + minimal salts + 0.5% rapeseed oil), incubation at 60°C; and
- inoculation from individual colonies into liquid medium and continuation as above.

Colonies on solid medium plates which had been identified as lipase-producers, either as small colonies surrounded by large clearing zones (extracellular lipase producers) or as spreading colonies with small clearing zones (intracellular lipase producers), were inoculated into the liquid medium at each round in the procedure.

12 new lipase-producing thermophilic strains were isolated, as detailed in Table 2.

**Table 2 Lipase-producing thermophilic isolates**

| Isolate | Spread of colony | Size of clearing zones |
|---|---|---|
| N2a | ++ | ++ |
| N2b | +++ | + |
| N2c | +++ | + |
| N12 | + | + |
| N13a | -* | + |

(continued)

| Isolate | Spread of colony | Size of clearing zones |
|---|---|---|
| N13b | -* | ++ |
| WM2 | ++ | + |
| WM3 | -* | ++ |
| OK1 | + + | + |
| OK2 | +++ | ++ |
| HG3a | + | ++ |
| HG3b | +++ | + |
| * These colonies are small, round and compact. They are, therefore, not considered to be "spread" and so are considered to be extracellular lipase producers. | | |

*iii) Assays for lipase activity*

The initial screening of colonies for lipase activity was based on the appearance of clearing zones when grown on plates containing rapeseed oil.

A colorimetric assay was used for the quantitative comparison of lipase activity. This assay is based on the formation of a coloured product (4-nitrophenol) by hydrolysis of the 4-nitrophenyl ester of a fatty acid. Since vegetable oils contain triglyceride esters of long chain fatty acids (C16 - C22), 4-nitrophenyl palmitate (C16) was selected as the most suitable commercially-available substrate.

The spectrophotometric assay is based on the hydrolysis of a fatty acid p-nitrophenyl ester to produce a nitrophenoxide ion, resulting in an increase in absorbance at 410 nm. In order to differentiate between lipase and esterase activity, p-nitrophenylpalmitate (C16) was used as substrate.

A stock solution (solution A) containing 16.5 mM p-nitrophenylpalmitate (p-npp) in 2-propanol was prepared. Solution B contained 0.4% (v/v) Triton X-100, 0.1 % (w/v) Gum Arabic in 50 mM Tris buffer pH 8 (equivalent to pH 7 at 60°C). Solutions A and B were first heated to 60°C, then solution B (810μl) was added to a spectrophotometer cuvette followed by 90μl of solution A. After mixing, the enzyme sample (100μl) was added and the change in $A_{410}$ recorded over a 2 min period.

Initially the assay was established with a commercially-available lipase isolated from *Candida rugosa* (Sigma Aldrich, product no. L1754 (Amano lipase AY30)) *C. rugosa* lipase is not regarded as thermostable. It has been shown (Matsumoto, M and Ohashi, K (2003) Biochem. Eng. J. 14 75-77) that the activity of this lipase when in free form declines rapidly at pH = 7.2 and 50°C and even more rapidly at 80°C. Table 3 shows that lipase activity was detectable at 37°C and 60°C although, as expected for a mesophilic enzyme, the activity was reduced at high temperature. Importantly, the background rate of substrate hydrolysis in the absence of enzyme was minimal.

**Table 3 Spectrophotometric assay of lipase activity**

| Enzyme Sample | Assay Temperature (°C) | Rate ($A_{410}$ $min^{-1}$) |
|---|---|---|
| Phosphate buffer pH7.2 | 37 | 0.002 |
| Phosphate buffer pH7.2 | 60 | 0.001 |
| Tris buffer pH8 (at 25°C) | 60 | 0.002 |
| *C. rugosa* lipase (10μg) | 37 | 0.576 |
| *C. rugosa* lipase (10μg) | 60 | 0.138 |

Lower reaction rates were observed when the assay was repeated with 4-nitrophenyl stearate (C18) as a substrate, confirming the use of 4-nitrophenyl palinitate as the most suitable substrate for activity screens on lipase-producing bacterial strains.

The assay described above was used for quantitative comparison of lipase activity in representative examples of the thermophilic strains and isolates described in Sections (i) and (ii). Table 4 shows that both extracellular and intracellular lipase activity was detectable in two of the seven strains tested and that a third strain showed low level intracellular activity.

**Table 4 Assay of lipase activity in thermophilic strains**

| Isolate | Cell pellet lipase activity (U/g wet cell mass) | Supernatant lipase activity (U/ml cell supernatant) |
|---|---|---|
| H2 | 0.15 | 0.0082 |
| G6 | 0.19 | 0.011 |
| F1 | Nd | Nd |
| F4b | Nd | Nd |
| N2b | 0.016 | Nd |
| N13a | Nd | Nd |
| OK1 | Nd | Nd |
| nd no activity detectable | | |

There is very limited correlation between the results of the lipase activity assays (Table 4) and the appearance of a clearing zone surrounding bacterial colonies grown on plates (Tables 1 & 2).

It is possible that 4-nitrophenyl palmitate may not be an appropriate substrate for all strains tested. In some cases, the p-nitrophenyl moiety is not well accepted by some enzymes in this type of colorimetric assay. For these reasons, this assay was not used in subsequent experiments.

**Transmethylation of vegetable oil**

*a) Validation of analytical method*

A gas chromatographic (gc) assay was developed to assess the suitability of various enzymes and cell preparations as catalysts for biodiesel production. The suitability of the assay was demonstrated using commercially-available lipases to produce fatty acid monoalkyl esters from rapeseed oil.

Hydrolysis of fatty acid triglycerides (i.e. vegetable oil) or synthetic substrates was used for initial screening of thermophilic strains for lipase activity. However, biodiesel production requires lipases that are active in non-aqueous conditions and are able to catalyse the transmethylation of triglycerides in the presence of methanol. The products of this reaction, fatty acid monoalkyl esters (FAMEs), can be analysed by gas chromatography (gc).

The gc method for detection of FAMEs was calibrated with commercial FAME standards (Sigma-Aldrich) and then tested by detection of the products of chemically-catalysed transmethylation of rapeseed oil. In order to prepare FAMEs by chemical catalysis, rapeseed oil (10mg) was weighed into a glass vial and methanol (1ml), hexane (1ml) and 2,2-dimethoxypropane (250μl) were added, followed by 100mg strongly acidic catalytic beads (Amberlyst 131; Sigma-Aldrich). The vial was incubated at 70°C for 10-15min and then allowed to cool. A further 1ml hexane and 1ml water were added and the vial was shaken vigorously. The layers were then allowed to separate so that the organic (upper) layer could be sampled for gc analysis of the FAME products.

As shown in Figure 2A, clear separation of FAMEs of chain length C16-C20 was achieved using calibration with commercial FAME standards. Figure 2B shows the production of FAMEs from rapeseed oil by the chemical procedure described above. In this case the main products are oleic acid (C18:1) and linoleic acid (C18:2) monoalkyl esters. This demonstrates that gc analysis is a suitable method for detection of FAMEs produced by lipase-catalysed transmethylation of rapeseed oil.

*b) Lipase-catalysed transmethylation - commercially available lipases*

Transmethylation reactions were performed in glass tubes containing 2ml hexane, 0.2mmol rapeseed oil, and 0.6mmol methanol. In initial trials, commercial lipases were used as a catalyst as detailed in Table 5. Catalyst (commercial immobilised lipase) was added and the tubes were incubated at 40°C for the commercial lipase from *C. rugosa,* for 16 h with continuous agitation. To extract the reaction products 1ml hexane (containing the internal standard C23:0 monoalkyl ester) and 1ml water were added and, after vigorous shaking, the upper organic hexane layer was sampled for gc analysis.

Samples were assayed for fatty acid monoalkyl ester (FAME) content by injecting 1μl of the hexane solution onto a BPX70 capillary gc column (15m x 0.25mm I.D., SGE Europe Ltd, UK). FAMEs were separated under isothermal conditions at 175°C for 6 min; both the injector and F.I.D. detector temperatures were 250°C. The column was calibrated with FAME reference standards (C16:0 - C20:1) and tricosenoic acid (C23:0) monoalkyl ester was included as internal standard for quantitation. FAME reference standards and internal standard were obtained from Sigma-Aldrich UK. All samples were assayed in triplicate.

Transesterification activity was expressed in gc units/mg enzyme, where

$$\text{Activity (gc units)} = [\text{sample C18:2 peak height}] / [\text{internal standard peak height}]$$

Comparison of the specific activity of commercial lipase preparations, expressed in gc units/mg protein, (Table 5) with the corresponding specific activities quoted by the enzyme supplier, expressed as μmol product formed/ min/ mg protein, forms the basis for a 'conversion factor', which was used to express the lipase specific activity results for fixed cells in standard units. Based on the results for the *C. antarctica* and *P. fluorescens* enzymes, the 'conversion factor' (gc units/standard Units) = 5. This factor was used to calculate the specific activity (Units/mg) values.

The different commercial lipase preparations showed a range of specific activities in the transmethylation reaction (Table 5), which reflects the differing specific activities of the enzymes as quoted by the supplier. In each case the gc trace showed the presence of FAMEs corresponding to each of the major fatty acid components of rapeseed oil. Figure 3 shows a representative trace of the products of the transmethylation reaction catalysed by a commercial lipase. This Figure shows that methylation is occurring in the presence of a 3:1 methanol:oil ratio with no sign of inhibition of the process and that good quantities of several FAMEs are being produced. In addition, by comparison with Figure 2B which shows a chemically-catalysed reaction, it can be seen that the the lipase-catalysed reaction is more successful, in that greater quantities of more FAMEs are produced.

**Table 5. Transmethylation activity of commercial lipases**

| Enzyme | Details | Reaction temperature (°C) | Activity (gc units) | Specific activity (gc units/ mg enzyme) |
|---|---|---|---|---|
| *Candida Antarctica* lipase | Fluka (Sigma Aldrich, St Louis, Missouri, USA), product no. 73940 (Novozym 435) Immobilised on BioChemika beads Specific activity 2.5 Units/mg Assayed at 40°C by hydrolysis of tributyrin | 37 | 91 | 14 |
| *Pseudomonas fluorescens* lipase | Fluka, product no. 62275 Immobilised in Sol-Gel-AK in sintered glass Specific activity 18.3 Units/g Assayed at 20°C by formation octyl laurate from octanol and lauric acid | 37 | 2.1 | 0.34 |
| *Bacillus stearothermop hilus* esterase | Fluka, product no. 46051 Specific activity 0.4 Units/mg Assayed at 65°C by hydrolysis of 4-nitrophenyl caproate | 60 | 0.5 | 0.08 |
| Reactions were incubated for 18 h at the temperature indicated.<br>Activity (gc units) = peak height C 18:2 (product) / peak height C23:0 (internal standard) | | | | |

*c) Lipase-catalysed transmethylation - lipases in cell preparations of thermophilic microorganisms*

The suitability of thermophilic microorganisms as biocatalysts for production of FAMEs was assessed using a selection of the strains described in sections (i) and (ii) above. Since the transmethylation reaction requires a non-aqueous environment, cell preparations were fixed prior to use as biocatalysts, either by treatment with acetone or by freeze-drying.

Transmethylation reactions were performed in glass tubes containing 2ml hexane, 0.2mmol rapeseed oil, and 0.6mmol methanol. Catalyst (fixed cells of experimental strains) was added and the tubes were incubated at 60°C for 16 h with continuous agitation. To extract the reaction products 1ml hexane (containing the internal standard C23:0 monoalkyl ester), and 1ml water were added and, after vigorous shaking, the upper organic hexane layer was sampled for gc analysis. Samples were assayed for FAME content (Figure 4A shows FAME standards and Figure 4B shows a trace of the products of the transmethylation reaction catalysed by the lipase isolated from strain F4b) and the activity of each lipase isolate was determined as described in section (b) above.

As shown in Table 6, the highest total activities were exhibited by acetone-fixed H2, G6 and F4b cells and freeze-dried H2 and G6 cells. However, when specific activities were calculated, acetone-fixed F4b and G6 and freeze-dried H2 preparations were the most active.

Activity was shown for all preparations of Montserrat strains up to a temperature of at least 70°C (data not shown). Activity in other cell preparations was demonstrated to at least 60-65°C, with results for some cell preparations shown in Table 6.

**Table 6. The activities of immobilised cells in transmethylation of rapeseed oil**

| | **Activity** (gc units) | **fixed cells added** (mg) | **Specific Activity** (gc units/mg cells) | **Specific Activity** (μmol product formed/ min/mg protein) |
|---|---|---|---|---|
| **Isolate** | **Acetone-fixed cells** | | | |
| OK1 | 0.07 | 1.5 | 0.05 | 0.01 |
| N2b | 0.29 | 3.2 | 0.09 | 0.02 |
| N13a | 0.00 | 3.0 | 0.00 | 0 |
| H2 | 2.29 | 3.9 | 0.59 | 0.12 |
| G6 | 1.27 | 1.5 | 0.85 | 0.17 |
| F4b | 1.68 | 1.3 | 1.29 | 0.26 |
| **Isolate** | **Freeze dried cells** | | | |
| OK1 | 0.00 | 4.9 | 0.00 | 0 |
| N2b | 0.37 | 5.2 | 0.07 | 0.01 |
| N13a | 0.00 | 4.0 | 0.00 | 0 |
| H2 | 2.07 | 1.5 | 1.38 | 0.28 |
| G6 | 1.39 | 1.4 | 0.99 | 0.20 |
| F4b | 0.29 | 0.6 | 0.48 | 0.1 |

**Identification of lipase producing colonies as *Bacillus* species**

The 16S gene of strains OK1, N2b, G6, N13a, H2 and F4b were amplified and sequenced, using primers:

AGR GTT TGA TCM TGG CTC AG (SEQ ID NO:9)
GKT ACC TTG TTA CGA CTT (SEQ ID NO:10).

PCR cycle:

94°C for 15 mins (1 cycle);
94°C for 30s, 50°C for 30s, 72°C for 2 mins (30 cycles)
72°C for 5 mins (1 cycle).

The sequences were analysed using ANGIS (http://www.angis.org.au/) and NCBI nucleotide-nucleotide BLAST (http://www.ncbi.nlm.nih.gov/BLAST/), using default parameter settings. The highest homology of the 16S gene from the strains in each case was as shown in Table 7:

**Table 7. Homology of 16S genre nucleotide sequence of test strains with 16S gene nucleotide sequence of known species**

| Strain | Best Match | Accession no (NCBI PubMed Entrez). |
|---|---|---|
| | | |
| OK1 | *Geobacillus thermoleovorans* | AY074879 |
| N2b | *Geobacillus kaustophilus* | AY608934 |
| G6 | *Bacillus sp.* | AY608943 |
| N13a | *Bacillus sp.* | AY608943 |
| H2 | *Geobacillus kaustophilus* | AY608934 |
| F4b | *Bacillus sp.* | AY608943 |
| PubMed Entrez accession information can be accessed at: http://www.ncbi.nlm.mh.gov/entrez/query.fcgi?db=PubMed | | |

**Identification of two novel lipases**

*a) PCR amplification of lipase gene from strains producing lipase*

Genomic DNA of strains OK1, N2b, G6, N13a, H2 and F4b was extracted (see Appendix 1) and the lipase gene from each strain was amplified using the following primers:

**BthFpET** AAA AAA CGG GAT CCA GCG GCA GTT TCA CGC GCCA (SEQ ID NO: 1)
**BthRpET** GAT AAT CCC TAA GCT TAA GGC CGC AAA CTC GCC (SEQ ID NO:2)

PCR cycling conditions:

95°C for 15 min (1 cycle)
95°C for 30s, 54°C for 30s, 72°C for 90s (30 cycles)
72°C for 9 min (1 cycle)

PCR conditions:

2ng genomic DNA used as template
Polymerase: Amplitaq Gold (Roche), 1U for 100μl reaction
Primers: final concentration 0.2μM
Nucleotides: final concentration 100μM each dNTPs
25μl reaction in 0.2ml tubes
PCR machine Perkin ELMER Gene Amp PCR system 2400 was used

*b) Cloning the lipase genes into the pET vector*

The DNAs were loaded on an agarose gel, electrophoresed, cut out, cleaned up using Qiagen Qiaquick Gel Extraction Kit (Qiagen, Crawley, UK; Cat no. 28704) and prepared for cloning into pET-26B (+) vector. The restriction sites *BamHI* and *HindIII on* both insert and vector were used for cloning.

Insert: PCR amplified lipase gene from strains OK1, N2b, G6, N13a, H2 and F4b Restriction: enzymes *BamHI* and *HindIII*

Cleaned up and checked the DNA on a gel Vector: pET-26b (+) vector (containing kanamycin resistance gene)

Restriction: enzymes *BamHI* and *HindIII*
Cleaned up and dephosphorylated
Cleaned up and checked the DNA on a gel

- Ligation (Rapid ligation kit, Roche)
- Transformation in to DH5α cells
- Plated out on LB + Kan plates

Transformation was carried out as follows:

a) Thaw cells at room temperature, aliquot 100 μl into 15 ml polypropylene tubes.
b) Add 1-5 μl plasmid, mix gently, incubate on ice for 30 minutes.
c) Heat pulse 42°C for exactly 30 seconds, transfer back onto ice, incubate 2min.
d) Add 0.9 ml SOB media + 20 μl 20% Glucose, outgrow 1 hour 37°C with shaking.
e) Plate onto LB+Kan Agar plates.

Many transformants for each ligation/ transformation were obtained. The presence of cloned inserts in these transformants was tested (3-5 transformants for each ligation) by colony PCR, in which cells are taken from a single colony and added directly to the PCR reaction (the reaction is incubated at 95°C for 15 mins before starting PCR). The cell debris may optionally be centrifuged out prior to this.

*c) Plasmid DNA preparation and sequencing*

For each ligation, two transformants that contained an insert of the right size were used to inoculate a liquid culture. The cells were grown overnight at 37°C and plasmid DNA of each transformant was prepared for sequencing.
Two primers, T7 promoter and T7 terminator, were used to sequence the cloned gene:

T7 promoter: TAATACGACTCACTATAGGG (SEQ ID NO:11)
T7 terminator: GCTAGTTATTGCTCAGCGG (SEQ ID NO:12)

After the sequencing reactions, the DNAs were submitted for sequencing to Macquarie University DNA analysis facility, Sydney, Australia:
(http://www.bio .mq.edu.au/centres/sequencer/)
Sequencing results were analysed in ANGIS. The DNA sequences encoding the lipase in each strain are shown in Figure 17, which shows an alignment of the sequences. The sequences for OK1, N2b and N13a were identical (SEQ ID NO:13), as were the sequences for G6 and F4b (SEQ ID NO: 14). Table 8 shows the sequence identity between the various sequences.

**Table 8. % sequence identity between DNA sequences from OK1, N2b, N13a, G6, F4b and H2**

| | **N2b** (SEQ ID NO: 13) | **G6** (SEQ ID NO: 14) | **N13a** (SEQ ID NO: 13) | **H2** (SEQ ID NO: 15) | **F4b** (SEQ ID NO: 14) |
|---|---|---|---|---|---|
| **OK1** (SEQ ID NO: 13) | 100 | 97 | 100 | 97 | 97 |
| **N2b** (SEQ ID NO: 13) | | 97 | 100 | 97 | 97 |
| **G6** (SEQ ID NO: 14) | | | 97 | 99 | 100 |
| **N13a** (SEQ ID NO: 13) | | | | 97 | 97 |
| **H2** (SEQ ID NO: 15) | | | | | 99 |

Strains OK1, N2b and N13a were found to have identical lipase amino acid sequences to one another, the sequence being shown in SEQ ID NO:3. Each of strains G6, H2 and F4b had lipase amino acid sequences which were identical to one another (sequence shown in SEQ ID NO:4) but which differed from the sequence of SEQ ID NO:3. A comparison of these sequences with that of *B. thermoleovorans* is shown in Figure 5, which shows the sequence similarity between SEQ ID NO:3, SEQ ID NO:4 and the *B. thermoleovaran* sequence (SEQ ID NO:5). Amino acid residues in SEQ ID NO:3 and SEQ ID NO:4 which differ from the amino acid sequence at the equivalent position in SEQ ID NO:5 are shown underlined and bold.

Similarly, SEQ ID NO:3 & SEQ ID NO:4 is also compared with the lipase sequences of G. *stearothermophilus* L1 (NCBI PubMed Entrez Accession no. AAC12257; SEQ ID NO:6; Figure 6), *G. thermoleovorans* NY (NCBI PubMed Entrez Accession no. ABC 48693; SEQ ID NO:7; Figure 7) and *Bacillus* sp. Tp10A.1 (NCBI PubMed Entrez Accession no. AAF63229; SEQ ID NO:8; Figure 8).

Table 9 shows a comparison of % sequence identity between the sequence of OK1 (SEQ ID NO:3) and F4b (SEQ ID NO:4) and SEQ ID NOs:6, 7 and 8.

**Table 9. % sequence identity between SEQ ID NOs: 3, 4, 5, 6, 7, 8**

| | F4b (SEQ ID NO:4) | OK1 (SEQ ID NO:3) |
|---|---|---|
| OK1 (SEQ ID NO:3) | 99% | / |
| Btherm (SEQ ID NO:5) | 98% | 99% |
| Gstear (SEQ ID NO:6) | 92% | 92% |
| Gtherm (SEQ ID NO:7) | 93% | 95% |
| Tp10A.1 (SEQ ID NO: 8) | 92% | 93% |

These sequence comparisons show that SEQ ID NO:3 and SEQ ID NO:4 differ from known lipase sequences. Although the differences do not occur in the active site or metal ion binding sites (as compared with the crystal structure of *G. stearothermophilus* L1, see Tyndall et al (2002) J. Mol. Biol. 323 859-869), they may have some influence on the interaction and rigidity of the secondary/tertiary structure of the enzyme. For example, this may occur in the "channel" that seems to lead towards the active site and may possibly exert some influence on the "flexible lid" mentioned by Tyndall *et al.*

**Characterisation of lipase enzymes**

The lipase genes of OK1 and F4b were cloned into pET-26b as outlined above. Plasmid DNA of OK1pET and F4b-pET was prepared and used for transformation into arabinose inducible BL21AI *Escherichia coli* (*E. coli*) competent cells (Invitrogen, Inchinnan Business Park, Paisley, UK; Cat no. C6070-03). These cells, when exposed to arabinose, express the T7 RNA polymerase gene, such that the lipase gene carried in the pET-26b vector is expressed. The cells were streaked out on LB-Agar plates and tested for their lipase activity.

The tributyrin plate assay was used for qualitative screening of the lipases. In this assay, tributyrin (a fatty acid ester) is hydrolysed to butyric acid and glycerol, both of which are water soluble. Hence, when an active lipase is present, a clear zone appears around the colony.

The lipolytic activity of the OK1 and F4b recombinant lipases was tested on LB-kan medium supplemented with 1% glycerol tributyrate. Cells were patched onto these plates and incubated at 37°C. The lipase expressed by *Bacillus* sp. Tp10A.1 (TPL; accession no. AAA63229) was used as a positive control. The hydrolysis activity of this lipase was described by Sunna et al in Enzyme and Microbiol. Tech. (2002) 31 472-476.

Cultures used:

1) OK1pET
2) F4bpET
3) positive control: TPLpET
4) negative control: pET vector

Plates: LB/agar + kanamycin plates
Once plated, cultures were grown overnight at 37°C for 16h.
Induction: Arabinose (3% w/w); LB/agarose (0.5% w/w) overlaid on colonies.
Once plated, cultures were grown at 37°C for 4h, followed by a period of time (see below) at a higher temperature.
Detection: Agarose (0.6%); tributyrin (5.5%); phosphate buffer 50mM; overlay. Incubation at room temperature until clearing zones became visible

Figures 9, 10 and 11 show lipase activity for all three lipases after 1.5h, 2.5h and 3.5h (respectively) exposure to 80°C. In each Figure, panel A is a photograph of the plate and panel B is a graphical representation of the location of the colonies (solid circles) and clear zones (dotted circles) on the corresponding plate. These results show that both OK1 and F4b lipases, as well as TPL, have lipolytic activity after 2.5h at 80°C. The lipolytic activity of OK1 is not detectable after 3.5h at 80°C whilst the activity of the F4b and TPL lipases is both still detectable after that time. In fact, activity for F4b was still detectable after 5.5h at 80°C (Figure 12).

The experiment was, therefore, repeated at 82°C. In this case, OK1 activity after 2h at 82°C was found to be not detectable (Figure 13). F4B activity was detectable after 2h (Figure 13) and 2.5h (Figure 14) at 82°C, but not after 4h at 82°C (Figure 15).

Similar results were obtained using a slightly different expression system. The lipase genes were clones into pPRoEXHTa vector and transformed into DHB10 competent cells.

Cultures used:

1) OK1pPRoEXHTa
2) F4b pPRoEXHTa
3) positive control: TPL pPRoEXHTa
4) negative control: pPRoEXHTa

Plates: LB/agar + ampicillin plates
Once plated, cells were grown overnight at 37°C.
Induction: Agarose (0.6%); IPTG (0.6mM); overlaid on colonies
Once plated, cells were grown at 37°C for 2 hours, followed by a period of time at 82°C.
Detection: Agarose (0.6%); tributyrin (10%); overlay
Incubation at room temperature until clearing zones became visible (approximately 18 hours).

The results for incubation at 82°C for 2.5 hours were as shown in Figure 14 for the previous expression system. In addition, Figure 16 shows that, where cells expressing F4b are incubated at 82°C for 3.5 hours, no lipase activity is detected. In fact, the same result was also obtained for incubation at 82°C for 2 hours 50 minutes, indicating that inactivation of F4b at 82°C occurs sometime between 2.5 hours and 2 hours 50 minutes.

Overall, the results demonstrate that small changes in temperature and incubation periods can have a significant effect on lipase enzyme stability and activity and that the lipases according to the invention are stable for longer periods at higher temperatures than has been reported for prior art lipases.

**Appendix I**

■ Freeze and thaw sample/cells (1-3 times)
- 80 °C for 10 min - 50°C for 3 min
■ resuspend in 1ml 50mM Tris/HCl pH 8 + 150 µl 0.25M EDTA pH 8

■ 37 °C for 20 min
■ + 120µl Lysozyme (10 mg/ml, + 1.4 µl Mutanolysin 1:10 in water (100 µg/µl)
■ mixed by hand
■ 70 °C for 20 min

■ + 70 µl RNAseA (RNase 10 mg/ml)
■ 37 °C for 20 min

■ + 57 µl *S. griseus* protease (40 mg/ml), + 70 µl 10% SDS
■ mixed by hand
■ 50°C for 20 min
■ Transfer to 15 ml tube (pouring)
■ bring volume up to 5ml with TE buffer and transfer to Phase Lock gel light (15 ml) with pipette
■ put 5ml phenol / CIAA 1:1 (25 phenol:24 chloroform: isoamyl alcohol) in first 15 ml tube, mix by hand and transfer to Phase Lock gel and mix gently
■ spin at 1500g , 5 min, 20 °C (Sigma cooling centrifuge)

■ transfer aqueous layer into new phase lock tube
■ add 5ml phenol / CIAA 1:1 (25:24:1) to same tube

- mix gently
- spin at 1500g, 5 min, 20 °C (Sigma cooling centrifuge)

- transfer aqueous layer into new phase lock tube
- ad 5ml CIAA 24:1 and mix gently
- spin at 1500 g, 5 min, 20 °C (Sigma cooling centrifuge)
- transfer to new 1.5 ml tube

- NaAc / EtOH precipitation
- + 1/10 vol 3M Na acetate pH 5 ; mix gently (55µl)
- + 2 vol cold EtOH abs. mix gently (1ml)
- room temperature 20 min

- spin at 5000 rpm, 30 min , 20 °C,
- discard supernatant

- wash pellet with 3ml EtOH 70%
- spin at 5000 rpm, 10 min, 20 °C, discard s/n
- re-spin at 5000 rpm, 5 min, 20 °C
- remove the rest of supernatant, dry on tissue
- dry pellet at 50°C covered with tissue for 5min

- resuspend pellet in TE buffer based on pellet size
- dissolve at room temperature 1 h, overnight at 4°C
- store DNA at 4 °C

Phase Lock Gel (light) is from Eppendorf (cat no 0032 005.209, UK Eppendorf based at Histon, Cambridge))

## Claims

1. An isolated lipase expressed by a *Bacillus* cell, wherein the lipase has detectable lipase activity at a temperature of 80°C for a period of at least 1.5 hours, wherein the lipase is not expressed by *Bacillus* sp. Tp10A.1, and wherein the lipase is produced by any one of *Bacillus* strains NCIMB41255 (N2b), NCIMB41256 (OK1) and NCIMB41257 (N13a) and comprises the amino acid sequence of SEQ ID NO:3.

2. An isolated lipase expressed by a *Bacillus* cell, wherein the lipase has detectable lipase activity at a temperature of 80°C for a period of at least 1.5 hours, wherein the lipase is not expressed by *Bacillus* sp. Tp10A.1, and wherein the lipase is produced by any one of *Bacillus* strains NCIMB41254 (F4b), NCIMB41258 (G6) and NCIMB41259 (H2), and wherein the lipase has 98.5% sequence homology to SEQ ID NO:4.

3. A lipase according to claim 2 having at least 99% sequence identity to SEQ ID NO:4.

4. A lipase according to claim 2 or 3 having at least 99.5% sequence identity to SEQ ID NO:4.

5. A lipase according to any of claims 2 to 4 which comprises the amino acid sequence of SEQ ID NO:4.

**6.** An isolated microorganism deposited as one of: NCIMB41255 (N2b), NCIMB41256 (OK1), NCIMB41257 (N13a), NCIMB41254 (F4b), NCIMB41258 (G6) or NCIMB41259 (H2).

**7.** A method of isolating a lipase expressed by a *Bacillus* cell, wherein the lipase has detectable lipase activity at a temperature of 80°C for a period of at least 1.5 hours, wherein the lipase is not expressed by *Bacillus* sp. Tp10A. 1, and wherein the lipase is produced by any one of *Bacillus* strains NCIMB41255 (N2b), NCIMB41256 (OK1) and NCIMB41257 (N13a) the method comprising:

a) determining whether the *Bacillus* cell is expressing a lipase;
b) determining whether said lipase has detectable lipase activity at a temperature of 80°C for a period of at least 1.5 hours; and
c) isolating said lipase,

wherein steps (b) and (c) may be carried out in any order.

## Patentansprüche

**1.** Isolierte, von einer *Bacillus*-Zelle exprimierte Lipase, wobei die Lipase bei einer Temperatur von 80°C wenigstens 1,5 Stunden lang nachweisbare Lipaseaktivität hat, wobei die Lipase nicht von *Bacillus* sp. Tp10A.1 exprimiert wird und wobei die Lipase von jedem der *Bacillus*-Stämme NCIMB41255 (N2b), NCIMB41256 (OK1) und NCIMB41257 (N13a) produziert wird und die Aminosäuresequenz von SEQ ID NO:3 umfasst.

**2.** Isolierte, von einer *Bacillus*-Zelle exprimierte Lipase, wobei die Lipase bei einer Temperatur von 80°C wenigstens 1,5 Stunden lang nachweisbare Lipaseaktivität hat, wobei die Lipase nicht von *Bacillus* sp. Tp10A.1 exprimiert wird und wobei die Lipase von jedem der *Bacillus*-Stämme NCIMB41254 (F4b), NCIMB41258 (G6) und NCIMB41259 (H2) produziert wird und wobei die Lipase 98,5 % Sequenzhomologie mit SEQ ID NO:4 hat.

**3.** Lipase nach Anspruch 2, die wenigstens 99 % Sequenzidentität mit SEQ ID NO:4 hat.

**4.** Lipase nach Anspruch 2 oder 3, die wenigstens 99,5 % Sequenzidentität mit SEQ ID NO:4 hat.

**5.** Lipase nach einem der Ansprüche 2 bis 4, die die Aminosäuresequenz von SEQ ID NO:4 umfasst.

**6.** Isolierter Mikroorganismus, hinterlegt als einer von: NCIMB41255 (N2b), NCIMB41256 (OK1), NCIMB41257 (N13a), NCIMB41254 (F4b), NCIMB41258 (G6) oder NCIMB41259 (H2).

**7.** Verfahren zum Isolieren einer von einer *Bacillus*-Zelle exprimierten Lipase, wobei die Lipase bei einer Temperatur von 80°C wenigstens 1,5 Stunden lang nachweisbare Lipaseaktivität hat, wobei die Lipase nicht von *Bacillus* sp. Tp10A.1 exprimiert wird und wobei die Lipase von jedem der *Bacillus*-Stämme NCIMB41255 (N2b), NCIMB41256 (OK1) und NCIMB41257 (N13a) produziert wird, wobei das Verfahren umfasst:

a) Bestimmen, ob die *Bacillus*-Zelle eine Lipase exprimiert;
b) Bestimmen, ob diese Lipase bei einer Temperatur von 80°C wenigstens 1,5 Stunden lang nachweisbare Lipaseaktivität hat; und
c) Isolieren dieser Lipase,

wobei die Schritte (b) und (c) in beliebiger Reihenfolge durchgeführt werden können.

## Revendications

**1.** Lipase isolée, exprimée par une cellule de *Bacillus,* la lipase ayant une activité de lipase détectable à une température de 80 °C pendant au moins 1,5 heure, la lipase n'étant pas exprimée par *Bacillus* sp. Tp10A.1, et la lipase étant produite par l'une quelconque des souches de *Bacillus* NCIMB41255 (N2b), NCIMB41256 (OK1) et NCIMB41257 (N13a) et comprenant la séquence d'acides aminés de SEQ ID N°3.

**2.** Lipase isolée, exprimée par une cellule de *Bacillus,* la lipase ayant une activité de lipase détectable à une température de 80 °C pendant au moins 1,5 heure, la lipase n'étant pas exprimée par *Bacillus* sp. Tp10A.1, et la lipase étant produite par l'une quelconque des souches de *Bacillus* NCIMB41254 (F4b), NCIMB41258 (G6) et NCIMB41259 (H2) et la lipase ayant 98,5 % d'homologie de séquence avec SEQ ID N°4.

**3.** Lipase selon la revendication 2, ayant au moins 99 % d'identité de séquence avec SEQ ID N°4.

**4.** Lipase selon la revendication 2 ou 3, ayant au moins 99,5 % d'identité de séquence avec SEQ ID N°4.

**5.** Lipase selon l'une quelconque des revendications 2 à 4, qui comprend la séquence d'acides aminés de SEQ ID N°4.

**6.** Organisme isolé déposé comme l'un parmi : NCIMB41255 (N2b), NCIMB41256 (OK1), NCIMB41257 (N13a), NCIMB41254 (F4b), NCIMB41258 (G6) ou NCIMB41259 (H2).

**7.** Procédé d'isolement d'une lipase exprimée par une cellule de *Bacillus,* dans lequel la lipase a une activité de lipase détectable à une température de 80 °C pendant au moins 1,5 heure, dans lequel la lipase n'est pas exprimée par *Bacillus* sp. Tp10A.1, et dans lequel la lipase est produite par l'une quelconque des souches de *Bacillus* NCIMB41255 (N2b), NCIMB41256 (OK1) et NCIMB41257 (N13a), le procédé comprenant les étapes consistant à :

a) déterminer si la cellule de *Bacillus* exprime une lipase ;
b) déterminer si ladite lipase a une activité de lipase détectable à une température de 80 °C pendant au moins 1,5 heure ; et
c) isoler ladite lipase,

dans lequel les étapes (b) et (c) peuvent être réalisées en ordre quelconque.

# Figure 1

**A**

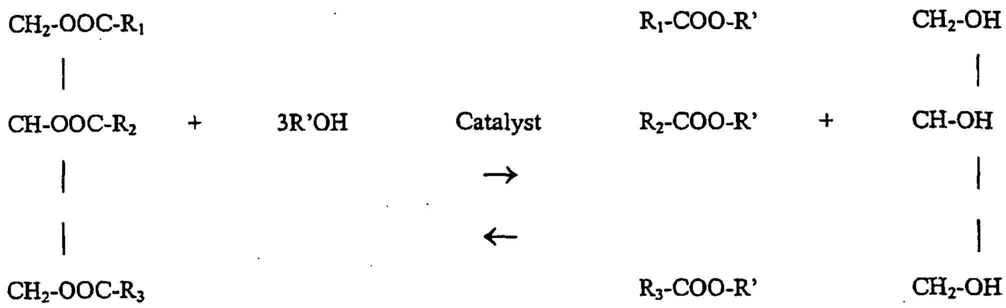


Triglyceride Alcohol Fatty acid esters Glycerol

**B**

1. Triglyceride (TG) + R'OH $\underset{}{\overset{\text{Catalyst}}{\rightleftarrows}}$ Diglyceride (DG) + $R'COOR_1$

2. Diglyceride (DG) + R'OH $\underset{}{\overset{\text{Catalyst}}{\rightleftarrows}}$ Monoglyceride (MG) + $R'COOR_2$

3. Monoglyceride (MG) + R'OH $\underset{}{\overset{\text{Catalyst}}{\rightleftarrows}}$ Glycerol (GL) + $R'COOR_3$

# Figure 2

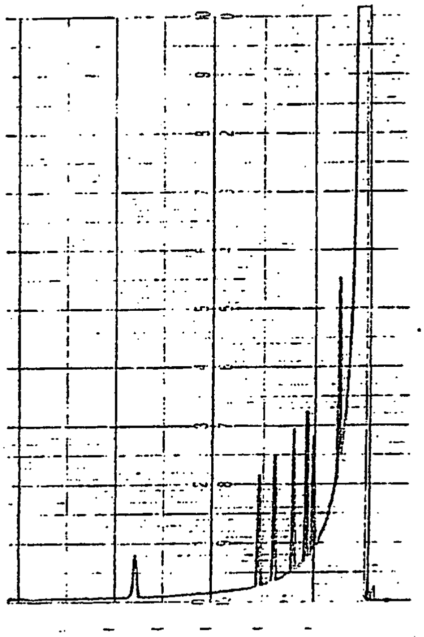

5

1 2 3 4 6 7

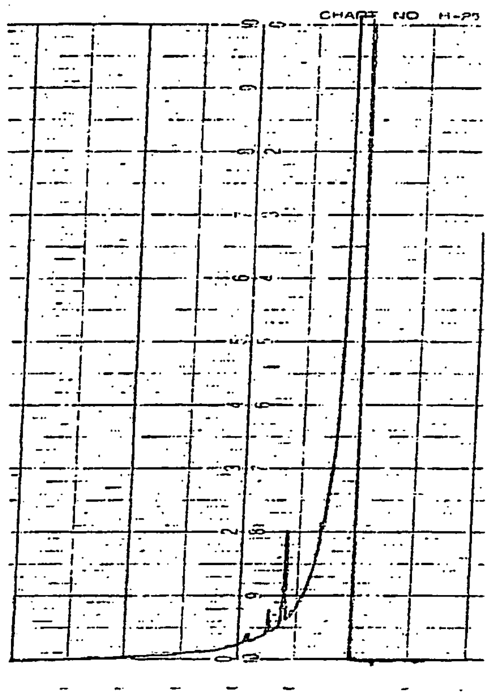

A

B

| | | | | |
|---|---|---|---|---|
| A | FAME standards: | 1 C16:0 | 2 C18:0 | 3 C18:1 |
| | | 4 C18:2 | 5 C18:3 | 6 C20:1 |
| | | 7 C23:0 | | |
| B | products of chemical transmethylation of rapeseed oil | | | |

# Figure 3

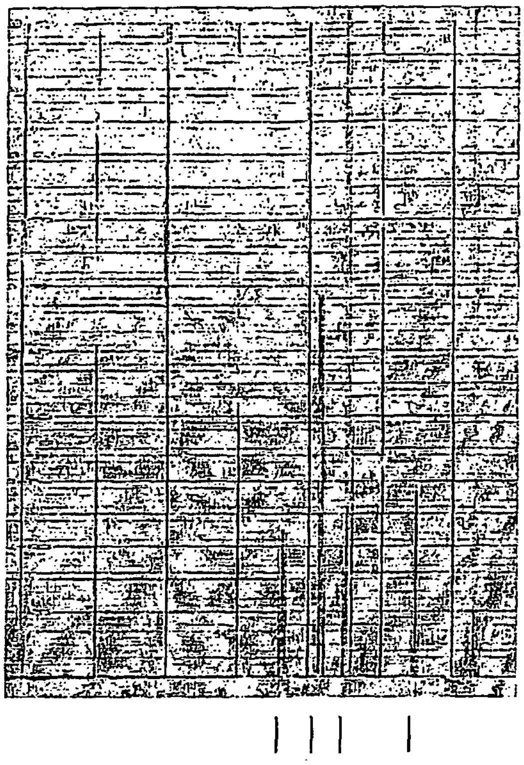


Representative trace for the products of lipase transmethylation of rapeseed oil

1 C16:0  2 C18:1  3 C18:2  4 C18:3

**Figure 4A**

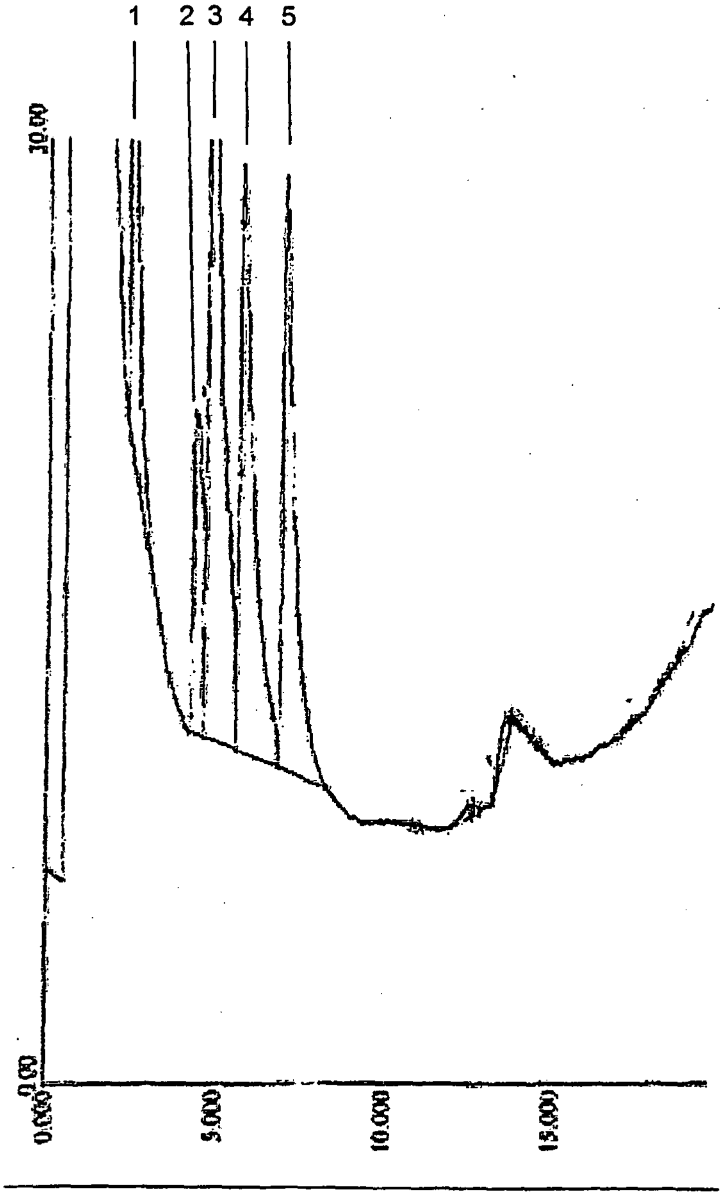


| Peak | Assignment |
|---|---|
| 1 | C16:0 |
| 2 | C18:0 |
| 3 | C18:1 |
| 4 | C18:2 |
| 5 | C18:3 |

**Figure 4B**

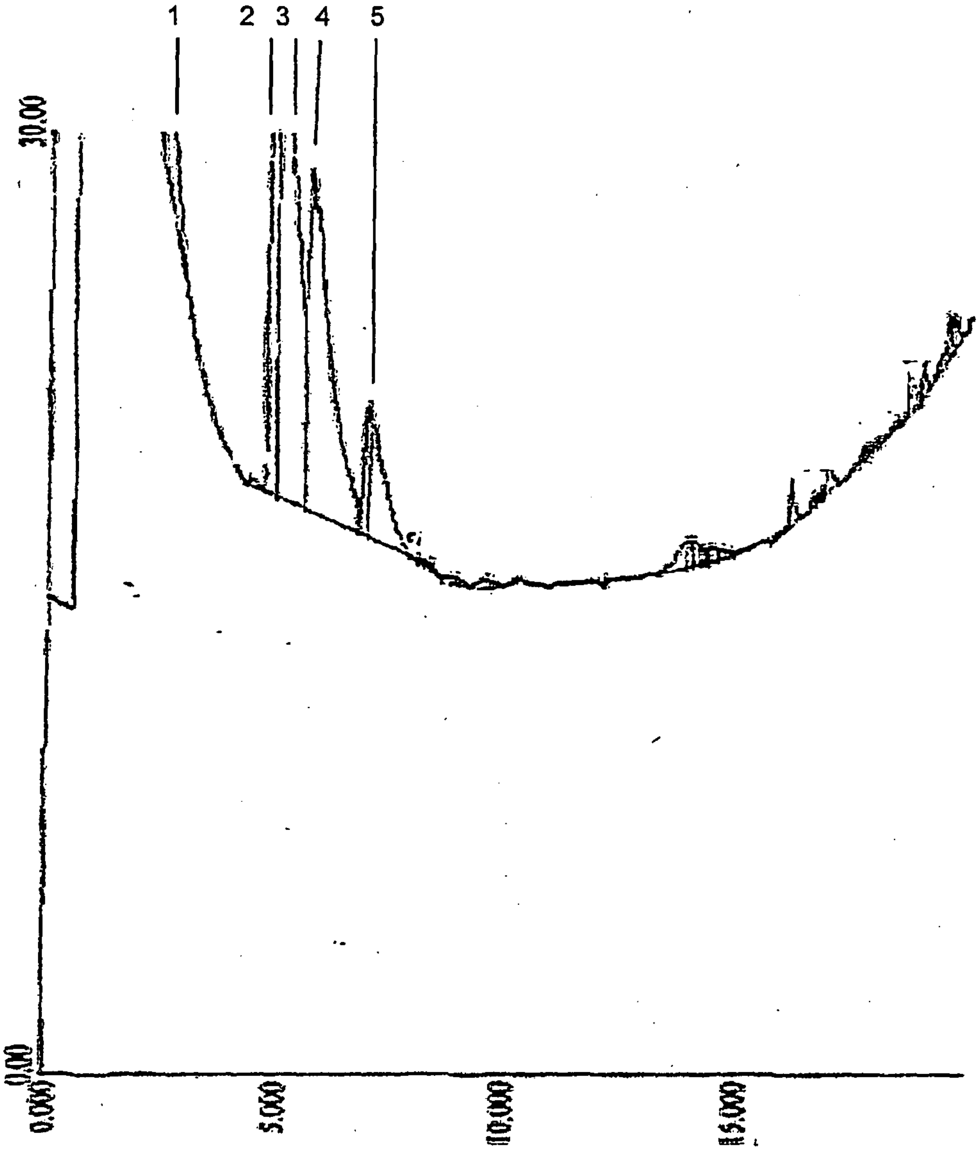


| Peak | Assignment |
|---|---|
| 1 | C16:0 |
| 2 | C18:0 |
| 3 | C18:1 |
| 4 | C18:2 |
| 5 | C18:3 |

# Figure 5

```
       1                                                                 60
OK1    ......AAVS RANDAPIVLL HGFTGWGREE MFGFKYWGGV RGDIEQWLND NGYRTYTLAV
N2b    .......... .......VLL HGFTGWGREE MFGFKYWGGV RGDIEQWLND NGYRTYTLAV
N13a   ........VS RANDAPIVLL HGFTGWGREE MFGFKYWGGV RGDIEQWLND NGYRTYTLAV
btherm ......AAVS RANDAPIVLL HGFTGWGREE MFGFKYWGGV RGDIEQWLND NGYRTYTLAV
G6     .....PAAVS RANDAPIVLL HGFTGWGREE MFGFKYWGGV RGDIEQWLND NGYRTYTLAV
F4b    FKNGDPAAVS RANDAPIVLL HGFTGWGREE MFGFKYWGGV RGDIEQWLND NGYRTYTLAV
H2     ......RQFS RANDAPIVLL HGFTGWGREE MFGFKYWGGV RGDIEQWLND NGYRTYTLAV

       61                                                               120
OK1    GPLSSNWDRA CEAYAQLVGG TVDYGAAHAA KHGHARFGRT YPGLLPELKR GGRIHIIAHS
N2b    GPLSSNWDRA CEAYAQLVGG TVDYGAAHAA KHGHARFGRT YPGLLPELKR GGRIHIIAHS
N13a   GPLSSNWDRA CEAYAQLVGG TVDYGAAHAA KHGHARFGRT YPGLLPELKR GGRIHIIAHS
btherm GPLSSNWDRA CEAYAQLVGG TVDYGAAHAA KHGHARFGRT YPGLLPELKR GGRIHIIAHS
G6     GPLSSNWDRA CEAYAQLVGG TVDYGAAHAA KHGHARFGRT YPGLLPELKR GGRVHIIAHS
F4b    GPLSSNWDRA CEAYAQLVGG TVDYGAAHAA KHGHARFGRT YPGLLPELKR GGRVHIIAHS
H2     GPLSSNWDRA CEAYAQLVGG TVDYGAAHAA KHGHARFGRT YPGLLPELKR GGRVHIIAHS

       121                                                              180
OK1    QGGQTARMLV SLLENGSQEE REYAKAHNVS LSPLFEGGHH VVLSVTTIAT PHDGTTLVNM
N2b    QGGQTARMLV SLLENGSQEE REYAKAHNVS LSPLFEGGHH VVLSVTTIAT PHDGTTLVNM
N13a   QGGQTARMLV SLLENGSQEE REYAKAHNVS LSPLFEGGHH VVLSVTTIAT PHDGTTLVNM
btherm QGGQTARMLV SLLENGSQEE REYAKAHNVS LSPLFEGGHH VMLSVTTIAT PHDGTTLVNM
G6     QGGQTARMLV SLLENGSQEE REYAKEHNVS LSPLFEGGHR FVLSVTTIAT PHDGTTLVNM
F4b    QGGQTARMLV SLLENGSQEE REYAKEHNVS LSPLFEGGHR FVLSVTTIAT PHDGTTLVNM
H2     QGGQTARMLV SLLENGSQEE REYAKEHNVS LSPLFEGGHR FVLSVTTIAT PHDGTTLVNM

       181                                                              240
OK1    VDFTDRFFDL QKAVLEAAAV ASNVPYTSQV YDFKLDQWGL RRQPGESFDH YFERLKRSPV
N2b    VDFTDRFFDL QKAVLEAAAV ASNVPYTSQV YDFKLDQWGL RRQPGESFDH YFERLKRSPV
N13a   VDFTDRFFDL QKAVLEAAAV ASNVPYTSQV YDFKLDQWGL RRQPGESFDH YFERLKRSPV
btherm VDFTDRFFDL QKAVLEAAAV ASNVPYTSQV YDFKLDQWGL RRQPGESFDH YFERLKRSPV
G6     VDFTDRFFDL QKAVLEAAAV ASNVPYTSQV YDFKLDQWGL RRQPGESFDH YFERLKRSPV
F4b    VDFTDRFFDL QKAVLEAAAV ASNVPYTSQV YDFKLDQWGL RRQPGESFDH YFERLKRSPV
H2     VDFTDRFFDL QKAVLEAAAV ASNVPYTSQV YDFKLDQWGL RRQPGESFDH YFERLKRSPV

       241                                                              300
OK1    WTSTDTARYD LSVSGAEKLN QWVQASPNTY YLSFATERTY RGALTGNYYP ELGMNAFSAV
N2b    WTSTDTARYD LSVSGAEKLN QWVQASPNTY YLSFATERTY RGALTGNYYP ELGMNAFSAV
N13a   WTSTDTARYD LSVSGAEKLN QWVQASPNTY YLSFATERTY RGALTGNYYP ELGMNAFSAV
btherm WTSTDTARYD LSVSGAEKLN QWVQASPNTY YLSFATERTY RGALTGNYYP ELGMNAFSAV
G6     WTSTDTARYD LSVSGAEKLN QWVQASPNTY YLSFSTERTY RGALTGNYYP ELGMNAFSAV
F4b    WTSTDTARYD LSVSGAEKLN QWVQASPNTY YLSFSTERTY RGALTGNYYP ELGMNAFSAV
H2     WTSTDTARYD LSVSGAEKLN QWVQASPNTY YLSFSTERTY RGALTGNYYP ELGMNAFSAV

       301                                                              360
OK1    VCAPFLGSYR NPTLGIDDRW LENDGIVNTV SMNGPKRGSS DRIVPYDGAL KKGVWNDMGT
N2b    VCAPFLGSYR NPTLGIDDRW LENDGIVNTV SMNGPKRGSS DRIVPYDGAL KKGVWNDMGT
N13a   VCAPFLGSYR NPTLGIDDRW LENDGIVNTV SMNGPKRGSS DRIVPYDGAL KKGVWNDMGT
btherm VCAPFLGSYR NPTLGIDDRW LENDGIVNTV SMNGPKRGSS DRIVPYDGAL KKGVWNDMGT
G6     VCAPFLGSYR NPTLGIDDRW LENDGIVNTV SMNGPKRGSS DRIVPYDGAL KKGVWNDMGT
F4b    VCAPFLGSYR NPTLGIDDRW LENDGIVNTV SMNGPKRGSS DRIVPYDGAL KKGVWNDMGT
H2     VCAPFLGSYR NPTLGIDDRW LENDGIVNTV SMNGPKRGSS DRIVPYDGAL KKGVWNDMGT

       361                                  398
OK1    YNVDHLEIIG VDPNPSFDIR AFYLRLAEQL ASLRP*A.
N2b    YNVDHLEIIG VDPNPSFDIR AFYLRLAEQL ASLRP*..
N13a   YNVDHLEIIG VDPNPSFDIR AFYLRLAEQL ASLRP*A.
btherm YNVDHLEIIG VDPNPSFDIR AFYLRLAEQL ASLRP*..
G6     YNVDHLEIIG VDPNPSFDIR AFYLRLAEQL ASLRP*A*
F4b    YNVDHLEIIG VDPNPSFDIR AFYLRLAEQL ASLRP*..
H2     YNVDHLEIIG VDPNPSFDIR AF........ ........
```

# Figure 6

```
OK1      ----------------------------AAVSRANDAPIVLLHGFTGWGREEMFGFKYWG 32
F4b      -----------------F-----KNGDPAAVSRANDAPIVLLHGFTGWGREEMFGFKYWG 38
Gstearo  MMKGCRVMVVLLGLWFVFGLSVPGGRTEAASPRANDAPIVLLHGFTGWGREEMLGFKYWG 60

OK1      GVRGDIEQWLNDNGYRTYTLAVGPLSSNWDRACEAYAQLVGGTVDYGAAHAAKHGHARFG 92
F4b      GVRGDIEQWLNDNGYRTYTLAVGPLSSNWDRACEAYAQLVGGTVDYGAAHAAKHGHARFG 98
Gstearo  GVRGDIEQWLNDNGYRTYTLAVGPLSSNWDRACEAYAQLVGGTVDYGAAHAANDGHARFG 120

OK1      RTYPGLLPELKRGGRIHIIAHSQGGQTARMLVSLLENGSQEEREYAKAHNVSLSPLFEGG 152
F4b      RTYPGLLPELKRGGRVHIIAHSQGGQTARMLVSLLENGSQEEREYAKEHNVSLSPLFEGG 158
Gstearo  RTYPGLLPELKRGGRVHIIAHSQGGQTARMLVSLLENGSQEEREYAKEHNVSLSPLFEGG 180

OK1      HHVVLSVTTIATPHDGTTLVNMVDFTDRFFDLQKAVLEAAAVASNVPYTSQVYDFKLDQW 212
F4b      HRFVLSVTTIATPHDGTTLVNMVDFTDRFFDLQKAVLEAAAVASNVPYTSQVYDFKLDQW 218
Gstearo  HRFVLSVTTIATPHDGTTLVNMVDFTDRFFDLQKAVLEAAAVASNAPYTSEIYDFKLDQW 240

OK1      GLRRQPGESFDHYFERLKRSPVWTSTDTARYDLSVSGAEKLNQWVQASPNTYYLSFATER 272
F4b      GLRRQPGESFDHYFERLKRSPVWTSTDTARYDLSVSGAEKLNQWVQASPNTYYLSFSTER 278
Gstearo  GLRREPGESFDHYFERLKRSPVWTSTDTARYDLSVPGAETLNRWVKASPNTYYLSFSTER 300

OK1      TYRGALTGNYYPELGMNAFSAVVCAPFLGSYRNPTLGIDDRWLENDGIVNTVSMNGPKRG 332
F4b      TYRGALTGNYYPELGMNAFSAVVCAPFLGSYRNPTLGIDDRWLENDGIVNTVSMNGPKRG 338
Gstearo  TYRGALTGNYYPELGMNAFSAIVCAPFLGSYRNAALGIDSHWLGNDGIVNTISMNGPKRG 360

OK1      SSDRIVPYDGALKKGVWNDMGTYNVDHLEIIGVDPNPSFDIRAFYLRLAEQLASLRP 389
F4b      SSDRIVPYDGALKKGVWNDMGTYNVDHLEIIGVDPNPSFDIRAFYLRLAEQLASLRP 395
Gstearo  SNDRIVPYDGTLKKGVWNDMGTYKVDHLEVIGVDPNPSFNIRAFYLRLAEQLASLRP 417
```

# Figure 7

```
OK1    ----------------------------AAVSRANDAPIVLLHGFTGWGREEMFGFKYWG 32
F4b    -----------------F-----KNGDPAAVSRANDAPIVLLHGFTGWGREEMFGFKYWG 38
Gtherm -MKCCRVMFVLLGLWLVFGLSVPGGRAEAATSRANDAPIVLLHGFTGWGREEMFGFKYWG 59

OK1    GVRGDIEQWLNDNGYRTYTLAVGPLSSNWDRACEAYAQLVGGTVDYGAAHAAKHGHARFG 92
F4b    GVRGDIEQWLNDNGYRTYTLAVGPLSSNWDRACEAYAQLVGGTVDYGAAHAAKHGHARFG 98
Gtherm GVRGDIEQWLNDNGYRTYTLAVGPLSSNWDRACEAYAQLVGGTVDYGAAHAAKHGHARFG 119

OK1    RTYPGLLPELKRGGRIHIIAHSQGGQTARMLVSLLENGSQEEREYAKAHNVSLSPLFEGG 152
F4b    RTYPGLLPELKRGGRVHIIAHSQGGQTARMLVSLLENGSQEEREYAKEHNVSLSPLFEGG 158
Gtherm RTYPGLLPELKRGGRIHIIAHSQGGQTARMLVSLLENGSQEEREYAKAHNVSLSPLFEGG 179

OK1    HHVVLSVTTIATPHDGTTLVNMVDFTDRFFDLQKAVLEAAAVASNVPYTSQVYDFKLDQW 212
F4b    HRFVLSVTTIATPHDGTTLVNMVDFTDRFFDLQKAVLEAAAVASNVPYTSQVYDFKLDQW 218
Gtherm HHFVLSVTTIATPHDGTTLVNMVDFTDRFFDLQKAVLEAAAVASNAPYTSEIYDFKLDQW 239

OK1    GLRRQPGESFDHYFERLKRSPVWTSTDTARYDLSVSGAEKLNQWVQASPNTYYLSFATER 272
F4b    GLRRQPGESFDHYFERLKRSPVWTSTDTARYDLSVSGAEKLNQWVQASPNTYYLSFSTER 278
Gtherm GLRREPGESFDHYFERLKRSPVWTSTDTARYDLSVPGAETLNRWVKASPNTYYLSFSTER 299

OK1    TYRGALTGNYYPELGMNAFSAVVCAPFLGSYRNPTLGIDDRWLENDGIVNTVSMNGPKRG 332
F4b    TYRGALTGNYYPELGMNAFSAVVCAPFLGSYRNPTLGIDDRWLENDGIVNTVSMNGPKRG 338
Gtherm TYRGALTGNYYPEFGMNAFSAIVCAPFFGSYRNAALGIDSHWLENDGIVNTISMNGPKRG 359

OK1    SSDRIVPYDGALKKGVWNDMGTYNVDHLEIIGVDPNPSFDIRAFYLRLAEQLASLRP 389
F4b    SSDRIVPYDGALKKGVWNDMGTYNVDHLEIIGVDPNPSFDIRAFYLRLAEQLASLRP 395
Gtherm SSDRIVPYDGALKKGVWNDMGTYNVDHLEIIGVDPNPSFDIRAFYLRLAEQLASLRP 416
```

# Figure 8

```
OK1       -----------------------------AAVSRANDAPIVLLHGFTGWGREEMFGFKYW 31
F4b       -----------------------FKNGDPAAVSRANDAPIVLLHGFTGWGREEMFGFKYW 37
Tp10A.1   MMKCCRRVALVLLGLWLVFCLSVPGGRAEAAASRANDAPIVLLHGFTGWGREEMFGFKYW 60

OK1       GGVRGDIEQWLNDNGYRTYTLAVGPLSSNWDRACEAYAQLVGGTVDYGAAHAAKHGHARF 91
F4b       GGVRGDIEQWLNDNGYRTYTLAVGPLSSNWDRACEAYAQLVGGTVDYGAAHAAKHGHARF 97
Tp10A.1   GGVRGDIEQLLNAQGYRTYTLAVGPLSSNWDRACEAYAQLVGGTVDYGAAHAAKHGHARF 120

OK1       GRTYPGLLPELKRGGRIHIIAHSQGGQTARMLVSLLENGSQEEREYAKAHNVSLSPLFEG 151
F4b       GRTYPGLLPELKRGGRVHIIAHSQGGQTARMLVSLLENGSQEEREYAKEHNVSLSPLFEG 157
Tp10A.1   GRTYPGLLPELKRGGRVHIIAHSQGGQTARMLVSLLENGSKEEREYAKAHNVSLSPLFEG 180

OK1       GHHVVLSVTTIATPHDGTTLVNMVDFTDRFFDLQKAVLEAAAVASNVPYTSQVYDFKLDQ 211
F4b       GHRFVLSVTTIATPHDGTTLVNMVDFTDRFFDLQKAVLEAAAVASNVPYTSQVYDFKLDQ 217
Tp10A.1   GHNFVLSVTTIATPHDGTTLVNMVDFTDRFFDLQKAVLKAAAVASNVPYTSQVYDFKLDQ 240

OK1       WGLRRQPGESFDHYFERLKRSPVWTSTDTARYDLSVSGAEKLNQWVQASPNTYYLSFATE 271
F4b       WGLRRQPGESFDHYFERLKRSPVWTSTDTARYDLSVSGAEKLNQWVQASPNTYYLSFSTE 277
Tp10A.1   WGLRRQPGESFDQYFERLKQSPVWTSADTARYDLSVPGAEALNQWVQASPNTYYLSFATE 300

OK1       RTYRGALTGNYYPELGMNAFSAVVCAPFLGSYRNPTLGIDDRWLENDGIVNTVSMNGPKR 331
F4b       RTYRGALTGNYYPELGMNAFSAVVCAPFLGSYRNPTLGIDDRWLENDGIVNTVSMNGPKR 337
Tp10A.1   RTYRGALTGNYYPELGMNVFSAAVCAPFLGSYRNAALGIDDRWLENDGIVNTFSMNGPKR 360

OK1       GSSDRIVPYDGALKKGVWNDMGTYNVDHLEIIGVDPNPSFDIRAFYLRLAEQLASLRP 389
F4b       GSSDRIVPYDGALKKGVWNDMGTYNVDHLEIIGVDPNPSFDIRAFYLRLAEQLASLRP 395
Tp10A.1   GSTDRIVPYDGTLKKGVWNDMGTYNVDHLEVIGVDPNPLFDIHAFYLRLAEQLASLRP 418
```

**Figure 9**

**A**

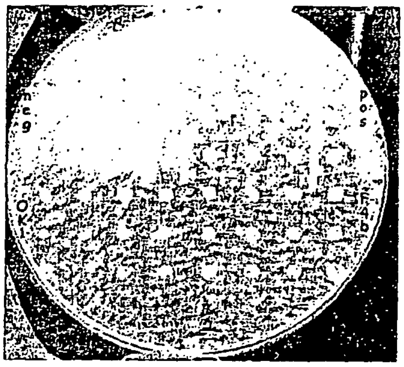

**B**

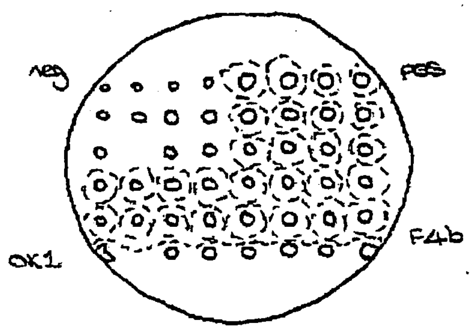


**Figure 10**

**A**

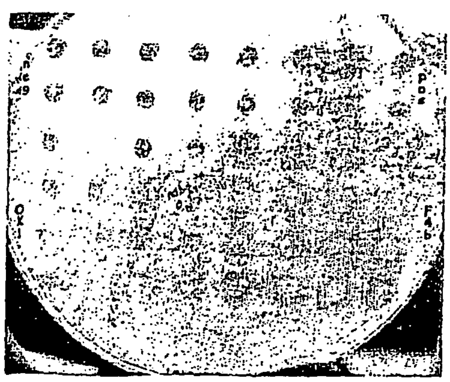

**B**

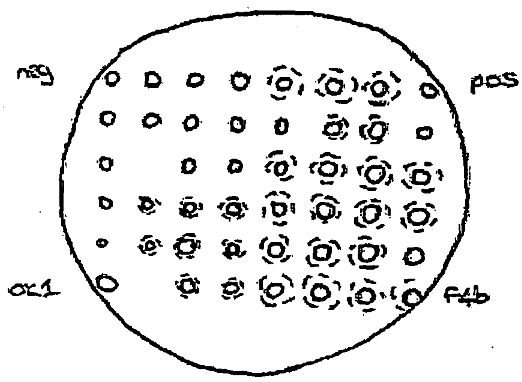

## Figure 11

A

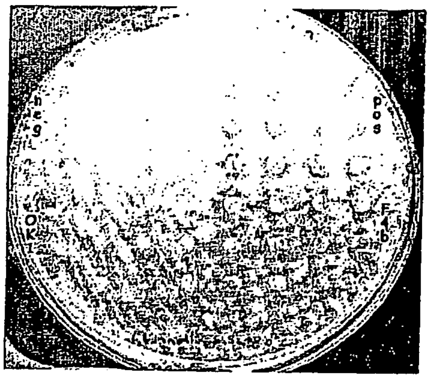

B

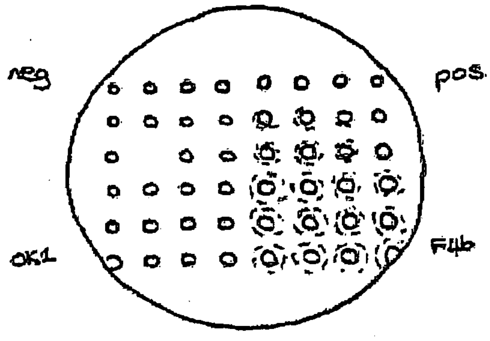


## Figure 12

A

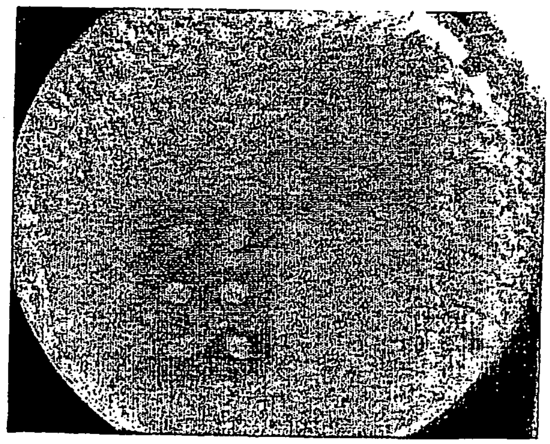

B

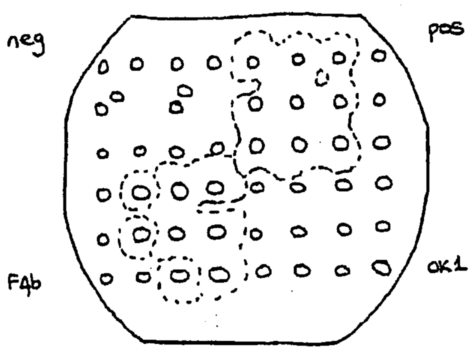

Figure 13

A B

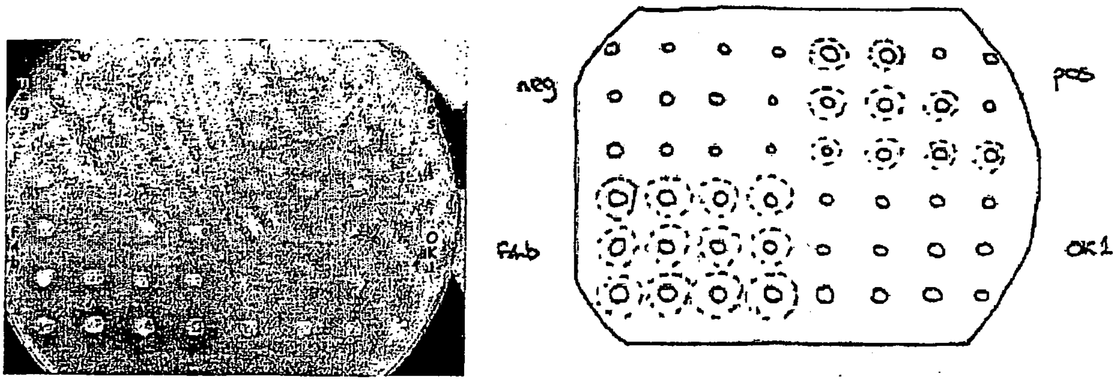


Figure 14

A B

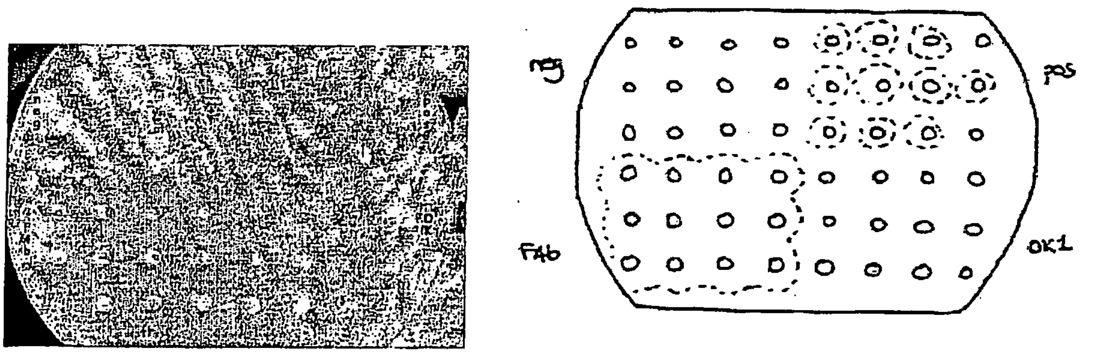

Figure 15

A

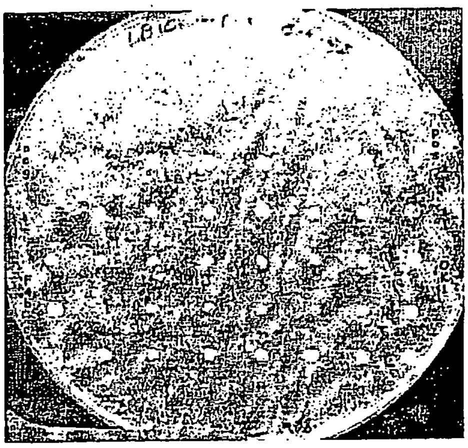

B

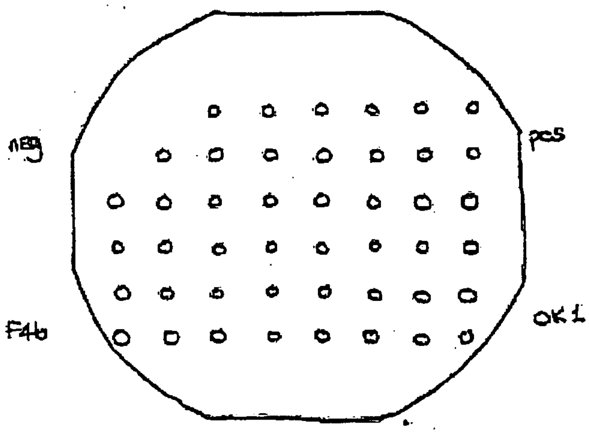


Figure 16

A

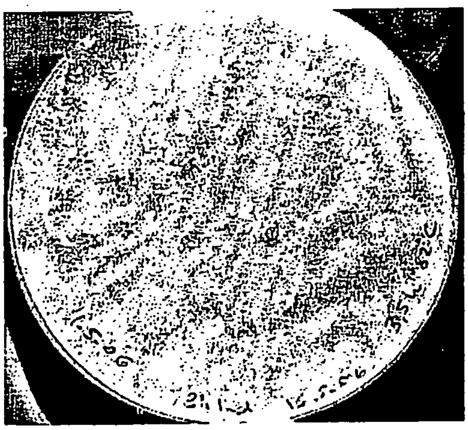

B

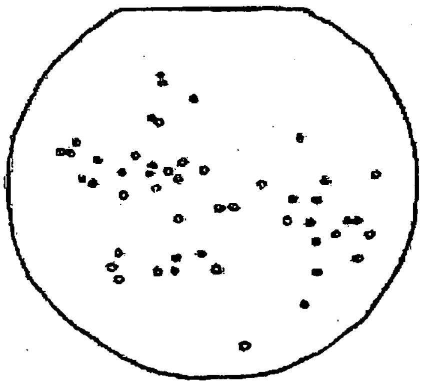

# Figure 17A

OK1, N2b, N13a = SEQ ID NO:13
G6, F4b = SEQ ID NO:14
H2 = SEQ ID NO:15

```
     1         11        21        31        41        51
OK1  atgcgccgattgtacTTCTCCATGGCTTTACTGGCTGGGGAAGAGAAGAAATGTTTGGGT
N2b  ATGCGCCGATTGTACTTCTCCATGGCTTTACTGGCTGGGGAAGAGAAGAAATGTTTGGGT
N13a ATGCGCCGATTGTACTTCTCCATGGCTTTACTGGCTGGGGAAGAGAAGAAATGTTTGGGT
G6   ATGCACcCaTCGTGCTTCTCCATGGTTTTACCGGCTGGGGAAGAGAAGAAATGTTTGGGT
F4b  ATGCACCCATCGTGCTTCTCCATGGTTTTACCGGCTGGGGAAGAGAAGAAATGTTTGGGT
H2   ATGCACCCATCGTGCTTCTCCATGGTTTTACCGGCTGGGGAAGAGAAGAAATGTTTGGGT

     61        71        81        91        101       111
OK1  TCAAGTACTGGGGCGGCGTgCGCGGCGATATCGAACAATGGCTGAACGACAACGGTTATC
N2b  TCAAGTACTGGGGCGGCGTGCGCGGCGATATCGAACAATGGCTGAACGACAACGGTTATC
N13a TCAAGTACTGGGGCGGCGTGCGCGGCGATATCGAACAATGGCTGAACGACAACGGTTATC
G6   TCAAGTACTGGGGCGGCGTGCGCGGCGATATCGAACAATGGCTGAACGACAACGGTTATC
F4b  TCAAGTACTGGGGCGGCGTGCGCGGCGATATCGAACAATGGCTGAACGACAACGGTTATC
H2   TCAAGTACTGGGGCGGCGTGCGCGGCGATATCGAACAATGGCTGAACGACAACGGTTATC

     121       131       141       151       161       171
OK1  GAACTTATACGCTGGCGGTCGGACCGCTCTCGAGCAACTGGGACCGGGCGTGTGAAGCGT
N2b  GAACTTATACGCTGGCGGTCGGACCGCTCTCGAGCAACTGGGACCGGGCGTGTGAAGCGT
N13a GAACTTATACGCTGGCGGTCGGACCGCTCTCGAGCAACTGGGACCGGGCGTGTGAAGCGT
G6   GAACTTATACGCTGGCGGTCGGACCGCTCTCGAGCAACTGGGACCGGGCGTGTGAAGCGT
F4b  GAACTTATACGCTGGCGGTCGGACCGCTCTCGAGCAACTGGGACCGGGCGTGTGAAGCGT
H2   GAACTTATACGCTGGCGGTCGGACCGCTCTCGAGCAACTGGGACCGGGCGTGTGAAGCGT

     181       191       201       211       221       231
OK1  ATGCTCAGCTTGTCGGCGGGACGGTCGATTATGGGGCAGCCCATGCGGCGAAGCACGGCC
N2b  ATGCTCAGCTTGTCGGCGGGACGGTCGATTATGGGGCAGCCCATGCGGCGAAGCACGGCC
N13a ATGCTCAGCTTGTCGGCGGGACGGTCGATTATGGGGCAGCCCATGCGGCGAAGCACGGCC
G6   ATGCTCAGCTTGTCGGCGGGACGGTCGATTATGGGGCAGCCCATGCGGCAAAGCACGGCC
F4b  ATGCTCAGCTTGTCGGCGGGACGGTCGATTATGGGGCAGCCCATGCGGCAAAGCACGGCC
H2   ATGCTCAGCTTGTCGGCGGGACGGTCGATTATGGGGCAGCCCATGCGGCAAAGCACGGCC

     241       251       261       271       281       291
OK1  ATGCGCGGTTTGGCCGCACTTATCCCGGCCTGTTGCCGGAATTGAAAAGGGGTGGCCGCA
N2b  ATGCGCGGTTTGGCCGCACTTATCCCGGCCTGTTGCCGGAATTGAAAAGGGGTGGCCGCA
N13a ATGCGCGGTTTGGCCGCACTTATCCCGGCCTGTTGCCGGAATTGAAAAGGGGtGGCCGCA
G6   ATGCGCGGTTTGGCCGCACGTATCCGGGGTTGCTGCCGGAATTGAAAAGAGGAGGCCGCG
F4b  ATGCGCGGTTTGGCCGCACGTATCCGGGGTTGCTGCCGGAATTGAAAAGAGGAGGCCGCG
H2   ATGCGCGGTTTGGCCGCACGTATCCGGGGTTGCTGCCGGAATTGAAAAGAGGAGGCCGCG

     301       311       321       331       341       351
OK1  TCCATATCATCGCCCACAGCCAAGGGGGGCAGACGGCCCGCATGCTTGTCTCGCTCCTAG
N2b  TCCATATCATCGCCCACAGCCAAGGGGGGCAGACGGCCCGCATGCTTGTCTCGCTCCTAG
N13a TCCATATCATCGCCCACAGCCAAGGGGGGCAGACGGCCCGCATGCTTGTCTCGCTCCTAG
G6   TCCATATCATCGCCCACAGCCAAGGAGGGCAGACGGCCCGTATGCTTGTCTCGCTCCTAG
F4b  TCCATATCATCGCCCACAGCCAAGGAGGGCAGACGGCCCGTATGCTTGTCTCGCTCCTAG
H2   TCCATATCATCGCCCACAGCCAAGGAGGGCAGACGGCCCGTATGCTTGTCTCGCTCCTAG
```

# Figure 17B

```
     361       371       381       391       401       411
OK1  AGAACGGAAGCCAAGAAGAGCGGGAGTACGCCAAGGCGCACAACGTGTCGTTGTCACCGT
N2b  AGAACGGAAGCCAAGAAGAGCGGGAGTACGCCAAGGCGCACAACGTGTCGTTGTCACCGT
N13a AGAACGGAAGCCAAGAAGAGCGGGAGTACGCCAAGGCGCACAACGTGTCGTTGTCACCGT
G6   AGAACGGAAGCCAAGAAGAGCGGGAGTACGCCAAGGAGCACAACGTGTCGTTGTCGCCGT
F4b  AGAACGGAAGCCAAGAAGAGCGGGAGTACGCCAAGGAGCACAACGTGTCGTTGTCGCCGT
H2   AGAACGGAAGCCAAGAAGAGCGGGAGTACGCCAAGGAGCACAACGTGTCGTTGTCGCCGT

     421       431       441       451       461       471
OK1  TGTTTGAAGGTGGACATCATGTTGTGTTGAGTGTGACGACCATCGCCACTCCTCATGACG
N2b  TGTTTGAAGGTGGACATCATGTTGTGTTGAGTGTGACGACCATCGCCACTCCTCATGACG
N13a TGTTTGAAGGTGGACATCATGTTGTGTTGAGTGTGACGACCATCGCCACTCCTCATGACG
G6   TGTTTGAAGGCGGACATCGTTTTGTGTTGAGCGTGAcGacCATcgCcACTCCTCATGACG
F4b  TGTTTGAAGGCGGACATCGTTTTGTGTTGAGCGTGACGACCATCGCCACTCCTCATGACG
H2   TGTTTGAAGGCGGACATCGTTTTGTGTTGAGCGTGACGACCATCGCCACTCCTCATGACG

     481       491       501       511       521       531
OK1  GGACGACGCTTGTCAACATGGTTGATTTCACCGATCGCTTTTTTGACTTGCAAAAAGCGG
N2b  GGACGACGCTTGTCAACATGGTTGATTTCACCGATCGCTTTTTTGACTTGCAAAAAGCGG
N13a GGACGACGCTTGTCAACATGGTTGATTTCACCGATCGCTTTTTTGACTTGCAAAAAGCGG
G6   GGACGACgCTTGTCAACATGGTTGATTTCACCGATCGCTTTTTTGACTTGCAAAAAGCGG
F4b  GGACGACGCTTGTCAACATGGTTGATTTCACCGATCGCTTTTTTGACTTGCAAAAAGCGG
H2   GGACGACGCTTGTCAACATGGTTGATTTCACCGATCGCTTTTTTGACTTGCAAAAAGCGG

     541       551       561       571       581       591
OK1  TGTTGGAAGCGGCGGCTGTCGCCAGCAACGTGCCGTACACGAGTCAAGTATACGATTTTA
N2b  TGTTGGAAGCGGCGGCTGTCGCCAGCAACGTGCCgTACACGAGTCAAGTATACGATTTTA
N13a TGTTGGAAGCGGCGGCTGTCGCCAGCAACGTGCCGTACACGAGTCAAGTATACGATTTTA
G6   TGTTGGAAGCGGCGGCTGTCGCCAGCAACGTGCCGTACACGAGTCAAGTATACGATTTTA
F4b  TGTTGGAAGCGGCGGCTGTCGCCAGCAACGTGCCGTACACGAGTCAAGTATACGATTTTA
H2   TGTTGGAAGCGGCGGCTGTCGCCAGCAACGTGCCGTACACGAGTCAAGTATACGATTTTA

     601       611       621       631       641       651
OK1  AGCTCGACCAATGGGGACTGCGCCGCCAGCCGGGTGAATCGTTCGACCATTATTTTGAAC
N2b  AGCTCGACCAATGGGGACTGCgCCGCCAGCCGGGTGAATCGTTCGACCATTATTTTGAAC
N13a AGCTCGACCAATGGGGACTGCGCCGCCAGCCGGGTGAATCGTTCGACCATTATTTTGAAC
G6   AGCTCGACCAATGGGGACTGCGCCGCCAGCCGGGTGAATCGTTCGACCATTATTTTGAAC
F4b  AGCTCGACCAATGGGGACTGCGCCGCCAGCCGGGTGAATCGTTCGACCATTATTTTGAAC
H2   AGCTCGACCAATGGGGACTGCGCCGCCAGCCGGGTGAATCGTTCGACCATTATTTTGAAC

     661       671       681       691       701       711
OK1  GGCTCAAGCGCTCCCCTGTTTGGACGTCGACAGATACCGCCCGCTACGATTTATCCGTTT
N2b  GGCTCAAGCGCTCCCCTGTTTGGACGTCGACAGATACCGCCCGCTACGATTTATCCGTTT
N13a GGCTCAAGCGCTCCCCTGTTTGGACGTCGACAGATACCGCCCGCTACGATTTATCCGTTT
G6   GGCTCAAGCGCTCCCCTGTCTGGACATCGACAGATACCGCCCGCTACGATTTATCCGTTT
F4b  GGCTCAAGCGCTCCCCTGTcTGGACATCGACAGATACCGCCCGCTACGATTTATCCGTTT
H2   GGCTCAAGCGCTCCCCTGTCTGGACATCGACAGATACCGCCCGCTACGATTTATCCGTTT
```

# Figure 17C

```
     721       731       741       751       761       771
OK1  CCGGAGCTGAGAAGTTGAATCAATGGGTGCAAGCAAGCCCGAATACGTATTATTTGAGCT
N2b  CCGGAGCTGAGAAGTTGAATCAATGGGTGCAAGCAAGCCCGAATACGTATTATTTGAGCT
N13a CCGGAGCTGAGAAGTTGAATCAATGGGTGCAAGCAAGCCCGAATACGTATTATTTGAGCT
G6   CCGGAGCTGAgAAgTTGAATCAATGGGTGCAAGCAAGCCCGAATACGTATTATTTGAGCT
F4b  CCGGAGCTGAGAAGTTGAATCAATGGGTGCAAGCAAGCCCGAATACGTATTATTTGAGCT
H2   CCGGAGCTGAGAAGTTGAATCAATGGGTGCAAGCAAGCCCGAATACGTATTATTTGAGCT

     781       791       801       811       821       831
OK1  TTGCCACAGAACGGACGTATCGCGGAGCGCTCACAGGCAACTATTATCCCGAACTCGGAA
N2b  TTGCCACAGAACGGACGTATCGCGGAGCGCTCACAGGCAACTATTATCCCGAACTCGGAA
N13a TTGCCACAGAACGGACGTATCGCGGAGCGCTCACAGGCAACTATTATCCCGAACTCGGAA
G6   TTTCTACCGAACGGACGTATCGCGGAGCGCTCACAGGCAACTATTATCCCGAACTCGGAA
F4b  TTTCTACCGAACGGACGTATCGCGGAGCGCTCACAGGCAACTATTATCCCGAACTCGGAA
H2   TTTCTACCGAACGGACGTATCGCGGAGCGCTCACAGGCAACTATTATCCCGAACTCGGAA

     841       851       861       871       881       891
OK1  TGAATGCATTCAGCGCGGTCGTATGCGCTCCGTTTCTCGGTTCGTACCGCAATCCGACGC
N2b  TGAATGCATTCAGCGCGGTCGTATGCGCTCCGTTTCTCGGTTCGTACCGCAATCCGACGC
N13a TGAATGCATTCAGCGCGGTCGTATGCGCTCCGTTTCTCGGTTCGTACCGCAATCCGACGC
G6   TGAATGCATTCAGCGCGGTCGTATGCGCTCCGTTTCTCGGTTCGTACCGCAATCCGACGC
F4b  TGAATGCATTCAGCGCGGTCGTATGCGCTCCGTTTCTCGGTTCGTACCGCAATCCGACGC
H2   TGAATGCATTCAGCGCGGTCGTATGCGCTCCGTTTCTCGGTTCGTACCGCAATCCGACGC

     901       911       921       931       941       951
OK1  TCGGCATTGACGACCGCTGGCTTGAAAACGATGGCATTGTCAATACGGTTTCCATGAACG
N2b  TCGGCATTGACGACCGCTGGCTTGAAAACGATGGCATTGTCAATACGGTTTCCATGAACG
N13a TCGGCATTGACGACCGCTGGCTTGAAAACGATGGCATTGTCAATACGGTTTCCATGAACG
G6   TCGGCATTGACGACCGCTGGCTTGAAAACGATGGTATTGTCAATACGGTTTCCATGAACG
F4b  TCGGCATTGACGACCGCTGGCTTGAAAACGATGGTATTGTCAATACGGTTTCCATGAACG
H2   TCGGCATTGACGACCGCTGGCTTGAAAACGATGGTATTGTCAATACGGTTTCCATGAACG

     961       971       981       991       1001      1011
OK1  GTCCAAAGCGTGGATCAAGTGATCGGATCGTACCGTATGACGGGGCGTTGAAAAAAGGGG
N2b  GTCCAAAGCGTGGATCAAGTGATCGGATCGTACCGTATGACGGGGCGTTGAAAAAAGGGG
N13a GTCCAAAGCGTGGATCAAGTGATCGGATCGTACCGTATGACGGGGCGTTGAAAAAAGGGG
G6   GTCCAAAGCGTGGATCAAGCGATCGGATCGTACCGTATGACGGGGCGTTGAAAAAAGGGG
F4b  GTCCAAAGCGTGGATCAAGCGATCGGATCGTACCGTATGACGGGGCGTTGAAAAAAGGGG
H2   GTCCAAAGCGTGGATCAAGCGATCGGATCGTCCCGTATGACGGGGCGTTGAAAAAAGGGG

     1021      1031      1041      1051      1061      1071
OK1  TTTGGAATGACATGGGAACGTACAATGTCGACCATTTGGAAATCATCGGCGTTGACCCGA
N2b  TTTGGAATGACATGGGAACGTACAATGTCGACCATTTGGAAATCATCGGCGTTGACCCGA
N13a TTTGGAATGACATGGGAACGTACAATGTCGACCATTTGGAAATCATCGGCGTTGACCCGA
G6   TTTGGAATGACATGGGAACGTACAATGTCGACCATTTGGAAATCATCGGCGTTGACCCGA
F4b  TTTGGAATGACATGGGAACGTACAATGTCGACCATTTGGAAATCATCGGCGTTGACCCGA
H2   TTTGGAATGACATGGGAACGTACAATGTCGACCATTTGGAAATCATCGGCGTTGACCCGA
```

# Figure 17D

```
     1081      1091      1101      1111      1121      1131
OK1  ATCCGTCATTTGATATTCGCGCCTTTTATTTGCGACTTGCCGAGCAGTTGGCGAGTTTGC
N2b  ATCCGTCATTTGATATTCGCGCCTTTTATTTGCGACTTGCCGAGCAGTTGGCGAGTTTGC
N13a ATCCGTCATTTGATATTCGCGCCTTTTATTTGCGACTTGCCGAGCAGTTGGCGAGTTTGC
G6   ATCCGTCATTTGATATTCGCGCCTTTTATTTGCGGCTTGCCGAGCAACTGGCGAGTTTGC
F4b  ATCCGTCATTTGATATTCGCGCCTTTTATTTGCGGCTTGCCGAGCAACTGGCGAGTTTGC
H2   ATCCGTCATTTGATATTCGCGCCTTTTATTTGCGGCTTGCCGAGCAACTGGCGAGTTTGC

     1141      1151
OK1  ggccttaagctt~~~~
N2b  GGCCTTAAGCTTAAGG
N13a GGCCTTAAGCT~~~~~
G6   GGCCTTAAGCTTAAGG
F4b  GGCCTTAAGCTTAAGG
H2   GGCCTTAAGCTT~~~~
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030032826 A
- US 5713965 A
- US 5935828 A
- WO 9004033 A
- EP 0709465 A
- US 20050204612 A1
- JP 6116585 B
- JP 1010994 A
- WO 0153511 A
- US 20060024789 A1
- EP 1624056 A

### Non-patent literature cited in the description


- **Fukuda, H et al.** *J. Bioscience & Bioengineering,* 2001, vol. 92 (5), 405-416
- **Abigor R D et al.** *Biochemical Society Transactions,* 2000, vol. 28, 979-981
- **Du W et al.** *Biotechnol. Appl. Biochem.,* 2003, vol. 38, 103-6
- **Hsu An C et al.** *Biotechnology and Applied Biochemistry,* 2002, vol. 36, 181-186
- Lipolytic Enzymes. Academic Press, 1974, 1-340
- Lipases. Elsevier, 1984, 1-420
- **Watanabe et al.** *JAOCS,* 2000, vol. 77, 355-360
- **Shimada et al.** *JAOCS,* 1999, vol. 76, 789-793
- **Tan et al.** *App. Biochem. Biotechnol.,* 2006, vol. 128, 109-116
- **Hirata et al.** *J. Biotechnol.,* 1990, vol. 14, 157-167
- **Basri et al.** *JAOCS,* 1997, vol. 74, 113-116
- **Goma-Donescu ; Legoy.** *JAOCS,* 1997, vol. 74, 1137-1143
- **Li ; Zhang.** *Protein Expression & Purification,* 2005, vol. 42, 153-159
- **Abdel-Fattah ; Gaballa.** *Microbiological Research,* 2006
- **Tyndall et al.** *J. Mol. Biol.,* 2002, vol. 323, 859-869
- **Cho et al.** *FEMS,* 2000, vol. 186, 235-238
- **Sunna et al.** *Enzyme & Microbial Technology,* 2002, vol. 31, 472-476
- **Atkinson et al.** *Extremophiles,* 2000, vol. 4, 305
- **Matsumoto, M ; Ohashi, K.** *Biochem. Eng. J.,* 2003, vol. 14, 75-77
- **Sunna et al.** *Enzyme and Microbiol. Tech.,* 2002, vol. 31, 472-476